(19) Europäisches Patentamt
European Patent Office
Office européen des brevets

(11) EP 1 992 325 A1

(12) **DEMANDE DE BREVET EUROPEEN**

(43) Date de publication:
**19.11.2008 Bulletin 2008/47**

(51) Int Cl.:
***A61K 8/06*** *(2006.01)* ***A61Q 1/00*** *(2006.01)*
***A61Q 19/00*** *(2006.01)*

(21) Numéro de dépôt: **08154891.9**

(22) Date de dépôt: **21.04.2008**

(84) Etats contractants désignés:
**AT BE BG CH CY CZ DE DK EE ES FI FR GB GR HR HU IE IS IT LI LT LU LV MC MT NL NO PL PT RO SE SI SK TR**
Etats d'extension désignés:
**AL BA MK RS**

(30) Priorité: **10.05.2007 FR 0754977**

(71) Demandeur: **L'Oreal**
**75008 Paris (FR)**

(72) Inventeurs:
- **Styczen, Patrice**
  **91190 Gif-sur-Yvette (FR)**
- **Ray, Xavier**
  **91580 Villeconin (FR)**
- **Gabin, Philippe**
  **91440 Bures sur Yvette (FR)**

(54) **Composition cosmétique sous forme de mousse sans cire**

(57) L'invention a pour objet une composition cosmétique sous forme de mousse comprenant une phase huileuse continue et au moins un agent structurant de la phase huileuse, ladite composition étant exempte de cire.

EP 1 992 325 A1

**Description**

La présente invention se rapporte à une composition sous forme de mousse comprenant une phase huileuse continue.

Les compositions selon l'invention peuvent être des compositions de maquillage ou de soin des matières kératiniques, en particulier de la peau, des lèvres, des cils, des sourcils ou des ongles. La composition peut être colorée ou non colorée.

Chaque composition peut être un fond de teint, un fard à joues ou à paupières, un produit anti-cerne, un blush, un rouge à lèvres, un baume à lèvres, un brillant à lèvres, un mascara, un eye-liner ou encore un produit de maquillage du corps ou de coloration de la peau.

La composition de soin peut être un produit de soin des cils, des lèvres, de soin de la peau du corps et du visage, notamment un produit solaire.

On connaît des compositions sous forme de mousse qui sont des émulsions huile dans eau ou cire dans eau comprenant une phase aqueuse continue.

Toutefois, ces compositions ne présentent pas toujours la brillance souhaitée par les consommatrices, notamment dans le cas des rouges à lèvres.

On connaît également du document US 2006/147390 des compositions comprenant des huiles, des cires et un gaz en une teneur minimale de 20%. Cependant, les cires seules ne permettent pas l'obtention d'une texture de mousse satisfaisante et présentant une brillance améliorée.

On cherche donc à obtenir des compositions qui présentent une texture originale, sous forme de mousse et qui présentent une brillance satisfaisante, via l'incorporation d'une certaine quantité d'huiles qui permettent d'apporter ces propriétés de brillance.

On cherche également à formuler des compositions comprenant une teneur élevée en huiles ou solvants organiques, volatiles ou non volatiles, de manière à améliorer la tenue et/ou la résistance à l'eau, au sébum et/ou les propriétés de non transfert desdites compositions.

En outre les compositions sous forme de mousse doivent présenter une bonne stabilité dans le temps, en particulier en termes d'homogénéité et d'aspect du produit.

La présente invention a pour but de proposer une autre voie de formulation pour une composition de revêtement des matières kératiniques qui présente une texture de mousse.

De façon plus précise, l'invention a pour objet une composition cosmétique sous forme de mousse comprenant une phase huileuse continue et au moins un agent structurant de la phase huileuse, ladite composition étant exempte de cire.

Par composition sous forme de mousse, on entend une composition comprenant une phase gazeuse (par exemple de l'air) sous forme de bulles, on parle encore de composition foisonnée.

La composition sous forme de mousse présente une texture légère, facile à prélever et à étaler sur les matières kératiniques.

L'invention a également pour objet un procédé de revêtement des matières kératiniques comprenant l'application sur lesdites matières kératiniques d'au moins une couche d'au moins une composition cosmétique sous forme de mousse comprenant une phase huileuse continue et au moins un agent structurant de la phase huileuse, ladite composition étant exempte de cire.

Le procédé de revêtement des matières kératiniques selon l'invention consiste à appliquer sur les matières kératiniques la composition sous forme de mousse, il se distingue des procédés de l'art antérieur en ce que la mousse ne se forme pas in situ sur les matières kératiniques, c'est-à-dire que la mousse ne se crée pas après application de ladite composition. En particulier, il ne s'agit pas d'une composition à expansion retardée qui est un système dans lequel un agent dit volatil est libéré ou formé dans la composition après que celle-ci ait été appliquée sur les matières kératiniques. Spécifiquement, les compositions à expansion retardée sont crées après exposition d'un gel à la pression atmosphérique, et/ou à un cisaillement et/ou à une température supérieure à la température ambiante.

L'invention a aussi pour objet un kit de maquillage et/ou de soin non thérapeutique des matières kératiniques comprenant :

- une composition sous forme de mousse comprenant une phase huileuse continue et au moins un agent structurant de la phase huileuse, ladite composition étant exempte de cire et
- un applicateur comportant au moins un élément d'application configuré pour appliquer la composition sur les matières kératiniques.

__Densité__

La composition sous forme de mousse présente notamment une densité ($d_{mousse}$) inférieure ou égale à 0,95,

de préférence inférieure ou égale à 0,9, mieux, inférieure ou égale à 0,8. La densité est de préférence supérieure ou égale à 0,2, et mieux supérieure ou égale à 0,3.
La densité est mesurée selon le protocole suivant : un récipient dont le volume Vo ($cm^3$) est connu avec une précision de $\pm$ 0.005 $cm^3$ (Vo étant de l'ordre de 10 $cm^3$), est pesé au moyen d'une balance de précision à $\pm$ 0.00005g. Sa masse est notée Mo (g). Ce récipient est rempli délicatement avec la mousse jusqu'au débordement du récipient. La surface du récipient est alors arasée avec une lame droite afin d'obtenir une surface de mousse parfaitement plane. On mesure alors la masse M (g) du récipient rempli de mousse.
La densité correspond au rapport entre la masse volumique de la composition calculée comme suit :

$$\rho_v (g/cm^3) = \frac{M - Mo}{Vo}$$

sur la masse volumique de l'eau (1 g/$cm^3$).
La composition mise en oeuvre dans le procédé selon l'invention présente une densité inférieure ou égale à 0,95, de préférence inférieure ou égale à 0,9, mieux, inférieure ou égale à 0,8. De préférence, la densité de la composition est supérieure ou égale à 0,2, et mieux supérieure ou égale à 0,3 .

## Taux de foisonnement

Le taux de foisonnement correspond à la quantité de gaz incorporée dans la composition.
Il peut être en particulier défini par la formule suivante :

$$\text{Taux de foisonnement} = \frac{d_{\text{avant foisonnement}} - d_{\text{mousse}}}{d_{\text{mousse}}} \text{X100}$$

La densité de la composition avant foisonnement est mesurée selon la méthode décrite plus haut pour la mousse.
La composition sous forme de mousse présente avantageusement un taux de foisonnement supérieur ou égal à 10%, et de préférence inférieur à 350%.
Le taux de foisonnement peut aller par exemple de 10% à 300%, de préférence de 30% à 250% et mieux de 40% à 200%.

## Taille des bulles :

La composition selon l'invention étant sous forme de mousse, elle comprend des bulles de gaz, avantageusement des bulles d'air.
En particulier, les bulles d'air de la composition présentent un diamètre moyen en nombre inférieur ou égal à 5 mm, allant par exemple de 0,1 $\mu$m à 5 mm, de préférence inférieur ou égal à 1 mm, et mieux inférieur ou égale à 0,8 mm.
Le diamètre moyen en nombre est déterminé de la façon suivante : la mousse est conditionnée en pot dès la fin de la préparation, lorsqu'elle est encore fluide. L'observation de la taille des bulles est effectuée en prenant une photo numérique de la surface du pot 24h après fabrication ou après ouverture du conditionnement après avoir arasé la surface , puis, à l'aide du logiciel de traitement d'image « Saisam », en comptant sur une surface d'environ 1$cm^2$ le nombre de bulles et en déterminant leur diamètre moyen en nombre.

## Extrait Sec

Les compositions selon l'invention peuvent présenter avantageusement une teneur en matière sèche (ou extrait sec) supérieure ou égale à 60 % en poids par rapport au poids total de la composition, de préférence supérieure ou égale à 65% en poids, mieux supérieure ou égale à 70% en poids, l'extrait sec pouvant aller jusqu'à 100% en poids par rapport au poids total de la composition.
La teneur en matière sèche, c'est à dire la teneur en matière non volatile, peut être mesurée de différentes manières, on peut citer par exemple les méthodes par séchage à l'étuve, les méthodes par séchage par exposition à un rayonnement infrarouge ainsi que les méthodes chimiques par titrage de l'eau selon Karl Fischer.

De préférence, la quantité de matière sèche, communément appelée « extrait sec » des compositions selon l'invention, est mesurée par échauffement de l'échantillon par des rayons infrarouges de 2 µm à 3,5 µm de longueur d'onde. Les substances contenues dans lesdites compositions qui possèdent une pression de vapeur élevée, s'évaporent sous l'effet de ce rayonnement. La mesure de la perte de poids de l'échantillon permet de déterminer « l'extrait sec » de la composition. Ces mesures sont réalisées au moyen d'un dessiccateur à infrarouges commercial LP16 de chez Mettler. Cette technique est parfaitement décrite dans la documentation de l'appareil fournie par Mettler.
Le protocole de mesure est le suivant :
On étale environ 1g de la composition sur une coupelle métallique. Celle-ci, après introduction dans le dessiccateur, est soumise à une consigne de température de 120 °C pendant une heure. La masse humide de l'échantillon, correspondant à la masse initiale et la masse sèche de l'échantillon, correspondant à la masse après exposition au rayonnement, sont mesurées au moyen d'une balance de précision.
La teneur en matière sèche est calculée de la manière suivante :

$$\text{Extrait Sec} = 100 \times (\text{masse sèche} / \text{masse humide}).$$

De préférence, la composition mise en oeuvre dans le procédé selon l'invention est une composition non rincée.

## I/ PHASE HUILEUSE CONTINUE

La composition selon l'invention comprend une phase huileuse (ou phase grasse liquide) continue.
Par phase huileuse, au sens de la demande, on entend une phase composée d'un ou plusieurs corps gras non aqueux liquides à température ambiante (25°C) et pression atmosphérique (760 mm de Hg), appelés aussi huiles ou solvants organiques, compatibles entre eux.
La phase huileuse continue peut représenter de 10 à 95 % du poids total de la composition, de préférence de 20 à 85% et mieux de 40 à 80 % en poids.
L'huile peut être choisie parmi les huiles volatiles et/ou les huile non volatiles, et leurs mélanges.
Par composition à phase continue huileuse, on entend que la composition présente une conductivité, mesurée à 25 °C, inférieure à 23 µS/cm (microSiemens/cm), la conductivité étant mesurée par exemple à l'aide d'un conductimètre MPC227 de Mettler Toledo et d'une cellule de mesure de conductivité Inlab730. La cellule de mesure est immergée dans la composition, de façon à éliminer les bulles d'air susceptibles de se former entre les 2 électrodes de la cellule. La lecture de la conductivité est faite dès que la valeur du conductimètre est stabilisée. Une moyenne est réalisée sur au moins 3 mesures successives.
Par " huile volatile", on entend au sens de l'invention une huile susceptible de s'évaporer au contact de la peau ou de la fibre kératinique en moins d'une heure, à température ambiante et pression atmosphérique. Le ou les solvants organiques volatils et les huiles volatiles de l'invention sont des solvants organiques et des huiles cosmétiques volatiles, liquides à température ambiante, ayant une pression de vapeur non nulle, à température ambiante et pression atmosphérique, allant en particulier de 0,13 Pa à 40 000 Pa ($10^{-3}$ à 300 mm de Hg), en particulier allant de 1,3 Pa à 13 000 Pa (0,01 à 100 mm de Hg), et plus particulièrement allant de de 1 ,3 Pa à 1300 Pa (0,01 à 10 mm de Hg).
Par "huile non volatile", on entend une huile restant sur la peau ou la fibre kératinique à température ambiante et pression atmosphérique au moins plusieurs heures et ayant notamment une pression de vapeur inférieure à $10^{-3}$ mm de Hg (0,13 Pa).
Ces huiles peuvent être des huiles hydrocarbonées, des huiles siliconées, des huiles fluorées, ou leurs mélanges.
On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en C8-C16 comme les isoalcanes en C8-C16 d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars' ou de Permetyls, les esters ramifiés en C8-C16 le néopentanoate d'iso-hexyle, et leurs mélanges. D'autres huiles hydrocarbonées volatiles comme les distillats de pétrole, notamment ceux vendus sous la dénomination Shell Solt par la société SHELL, peuvent aussi être utilisées. Selon un mode de réalisation, le solvant volatil est choisi parmi les huiles volatiles hydrocarbonées ayant de 8 à 16 atomes de carbone et leurs mélanges.
Comme huiles volatiles, on peut aussi utiliser les silicones volatiles, comme par exemple les huiles de silicones linéaires ou cycliques volatiles, notamment celles ayant une viscosité ≤ 8 centistokes (8 $10^{-6}$ $m^2/s$), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.
On peut également citer les huiles linéaires alkyltrisiloxanes volatiles de formule générale (I)

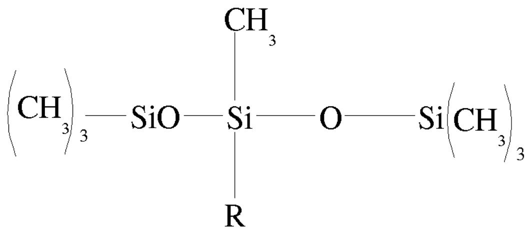

où R représente un groupe alkyle comprenant de 2 à 4 atomes de carbone et dont un ou plusieurs atomes d'hydrogène peuvent être substitués par un atome de fluor ou de chlore.
Parmi les huiles de formule générale (I), on peut citer :

le 3-butyl 1,1,1,3,5,5,5-heptaméthyl trisiloxane,
le 3-propyl 1,1,1,3,5,5,5-heptaméthyl trisiloxane, et
le 3-éthyl 1,1,1,3,5,5,5-heptaméthyl trisiloxane,

correspondant aux huiles de formule (I) pour lesquelles R est respectivement un groupe butyle, un groupe propyle ou un groupe éthyle.
On peut également utiliser des solvants volatils fluorés tels que le nonafluorométhoxybutane ou le perfluorométhylcyclopentane.
La composition peut comprendre au moins une huile non volatile, notamment choisie parmi les huiles hydrocarbonées et/ou siliconées et/ou fluorées non volatiles.
Comme huile hydrocarbonée non volatile, on peut notamment citer :

- les huiles hydrocarbonées d'origine végétale telles que les triesters d'acides gras et de glycérol dont les acides gras peuvent avoir des longueurs de chaînes variées de C4 à C24, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations Miglyol 810, 812 et 818 par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges;
- les esters de synthèse comme les huiles de formule R1COOR2 dans laquelle R1 représente le reste d'un acide gras linéaire ou ramifié comportant de 1 à 40 atomes de carbone et R2 représente une chaîne hydrocarbonée notamment ramifiée contenant de 1 à 40 atomes de carbone à condition que R1 + R2 soit $\geq$ 10, comme par exemple l'huile de Purcellin (octanoate de cétostéaryle), le myristate d'isopropyle, le palmitate d'isopropyle, le benzoate d'alcool en C12 à C15, le laurate d'hexyle, l'adipate de diisopropyle, l'isononanoate d'isononyle, le palmitate de 2-éthyl-hexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de diisostéaryle ; et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ;
- les carbonates,
- les acétales,
- les citrates,
- et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;

Les huiles fluorées utilisables dans l'invention sont notamment des huiles fluorosiliconées, des polyéthers fluorés, des silicones fluorées telles que décrit dans le document EP-A-847752.

Selon un mode de réalisation, la phase grasse contient une huile non volatile, de préférence hydrocarbonée, en particulier une huile ester. Cette huile ester peut être choisie parmi les esters des acides monocarboxyliques avec les monoalcools et polyalcools.

Avantageusement, ledit ester répond à la formule (I) suivante :

$$R_1\text{-CO-O-}R_2 \qquad (I)$$

où $R_1$ représente un radical alkyle linéaire ou ramifié de 1 à 40 atomes de carbone, de préférence de 7 à 19 atomes de carbone, comprenant éventuellement une ou plusieurs double liaisons éthyléniques, et éventuellement substitué.

$R_2$ représente un radical alkyle linéaire ou ramifié de 1 à 40 atomes de carbone, de préférence de 3 à 30 atomes de carbone et mieux de 3 à 20 atomes de carbone, comprenant éventuellement une ou plusieurs double liaisons éthyléniques, et éventuellement substitué.

Par « éventuellement substitué », on entend que $R_1$ et ou $R_2$ peuvent porter un ou plusieurs substituants choisis, par exemple, parmi les groupements comprenant un ou plusieurs hétéroatomes choisi parmi O, N et S, tels que amino, amine, alcoxy, hydroxyle.

De préférence, le nombre total d'atomes de carbone de $R_1$ + $R_2$ est $\geq 9$.

$R_1$ peut représenter le reste d'un acide gras, de préférence supérieur, linéaire ou, de préférence ramifié comprenant de 1 à 40 et mieux de 7 à 19 atomes de carbone et $R_2$ peut représenter une chaîne hydrocarbonée linéaire ou de préférence ramifiée contenant de 1 à 40, de préférence de 3 à 30 et mieux de 3 à 20 atomes de carbone. De nouveau, de préférence, le nombre d'atomes de carbone de $R_1 + R_2 \geq 9$.

Des exemples des groupes $R_1$ sont ceux dérivés des acides gras choisis dans le groupe constitué des acides acétique, propionique, butyrique, caproïque, caprylique, pélargonique, caprique, undécanoïque, laurique, myristique, palmitique, stéarique, isostéarique, arachidique, béhénique, oléique, linolénique, linoléïque, éléostéarique, arachidonique, érucique, et de leurs mélanges.

Des exemples d'esters sont, par exemple, l'huile de purcellin (octanoate de cétostéaryle), l'isononanoate d'isononyle, le myristate d'isopropyle, le palmitate d'éthyl-2-hexyle, le stéarate d'octyl 2-dodécyle, l'érucate d'octyl 2-dodécyle, l'isostéarate d'isostéaryle, et les heptanoates, octanoates, décanoates ou ricinoléates d'alcools ou de polyalcools, par exemple d'alcools gras.

Avantageusement, les esters sont choisis parmi les composés de la formule (I) ci-dessus, dans laquelle $R_1$ représente un groupe alkyle linéaire ou ramifié non substitué, comprenant éventuellement une ou plusieurs double liaisons éthyléniques, de 1 à 40 atomes de carbone, de préférence de 7 à 19 atomes de carbone, et $R_2$ représente un groupe alkyle linéaire ou ramifié non substitué, comprenant éventuellement une ou plusieurs double liaisons éthyléniques, de 1 à 40 atomes de carbone, de préférence de 3 à 30 atomes de carbone, et mieux de 3 à 20 atomes de carbone.

De préférence, $R_1$ est un groupe alkyle ramifié non substitué de 4 à 14 atomes de carbone, de préférence de 8 à 10 atomes de carbone et $R_2$ est un groupe alkyle ramifié non substitué de 5 à 15 atomes de carbone, de préférence de 9 à 11 atomes de carbone. De préférence dans la formule (I), $R_1$-CO- et $R_2$ ont le même nombre d'atomes de carbone et dérivent du même radical, de préférence alkyle ramifié non substitué, par exemple isononyle, c'est-à-dire que avantageusement la molécule d'huile ester est symétrique.

L'huile ester sera choisie, de préférence, parmi les composés suivants :

- l'isononanoate d'isononyle,
- l'octanoate de cétostéaryle,
- le myristate d'isopropyle,
- le palmitate d'éthyl-2-hexyle,
- le stéarate d'octyl 2-dodécyle,
- l'érucate d'octyl 2-dodécyle,
- l'isostéarate d'isostéaryle.

La phase huileuse peut comprendre de 0,5 à 100 % en poids, en particulier de 1 à 80 %, notamment de 2 à 50 % et plus particulièrement de 2 à 40 % de la ou des huiles esters.

**II/ AGENT STRUCTURANT**

L'agent structurant de la phase huileuse est avantageusement choisi parmi les agents structurants polymériques.

Par polymère, on entend au sens de l'invention un composé ayant au moins 2 motifs de répétition, de préférence au moins 3 motifs de répétition et mieux encore 10 motifs de répétition.

L'agent structurant polymérique présente avantageusement une masse moléculaire moyenne en poids (ou poids moléculaire) supérieure ou égale à 4000, mieux supérieure ou égale à 5000, et encore mieux, supérieure ou égale à 6000, pouvant aller jusqu'à 500 000.

Les agents structurants polymériques peuvent être notamment choisis parmi :

A/ les polymères siliconés comportant au moins un motif comprenant :

1) des polyorganosiloxanes comportant au moins deux groupes capables d'établir des interactions hydrogène, ces deux groupes étant situés dans la chaîne du polymère, et/ou
2) des polyorganosiloxanes comportant au moins deux groupes capables d'établir des interactions hydrogène, ces deux groupes étant situés sur des greffons ou ramifications,

lesdits groupes capables d'établir des interactions hydrogène sont choisis parmi les groupes ester, amide, sulfonamide, carbamate, thiocarbamate, urée, uréthane, thiourée, oxamido, guanidino, biguanidino et leurs combinaisons.
B/ les polymères de polyamide comportant un squelette polymérique ayant des motifs répétitifs hydrocarbonés pourvus d'au moins un motif amide non pendant, et au moins une chaîne grasse pendante et/ou au moins une chaîne grasse terminale éventuellement fonctionnalisées, comprenant au moins 4 atomes de carbone et étant liées à ces motifs hydrocarbonés,
C/ les copolymères d'oléfine,
D/ les organopolysiloxanes élastomériques partiellement ou totalement réticulés, de structure tridimensionnelle,
E/ les polymères semi-cristallins,
F/ les esters de dextrine et d'acide gras,

et leurs mélanges.

Le ou les agents structurants peu(ven)t représenter de 0,1 à 60 % en poids par rapport au poids total de la composition, de préférence de 0,5 à 50 % et de façon encore plus préférée de 1 à 40 % en poids.

La quantité en structurant de phase huileuse peut être ajustée par l'homme du métier en fonction des propriétés de structuration desdits agents.

**A/ Polymères siliconés**

Les polymères siliconés de la composition sont de préférence solides à la température ambiante (25°C) et pression atmosphérique (760 mm de Hg).

Par polymère, on entend au sens de l'invention un composé ayant au moins 2 motifs de répétition, de préférence au moins 3 motifs de répétition et mieux encore 10 motifs de répétition.

Les polymères siliconés utilisés comme agents structurants dans la composition de l'invention sont des polymères du type polyorganosiloxane comme par exemple ceux décrits dans les documents US-A-5 874 069, US-A-5,919,441, US-A-6,051,216 et US-A-5,981,680. Selon l'invention, les polymères utilisés comme agent structurant peuvent appartenir aux deux familles suivantes :

1) des polyorganosiloxanes comportant au moins deux groupes capables d'établir des interactions hydrogène, ces deux groupes étant situés dans la chaîne du polymère, et/ou
2) des polyorganosiloxanes comportant au moins deux groupes capables d'établir des interactions hydrogène, ces deux groupes étant situés sur des greffons ou ramifications.

Les groupes capables d'établir des interactions hydrogène peuvent être choisis parmi les groupes ester, amide, sulfonamide, carbamate, thiocarbamate, urée, uréthane, thiourée, oxamido, guanidino, biguanidino et leurs combinaisons.

a) Selon une première variante, les polymères siliconés sont des polyorganosiloxane tels que définis ci-dessus et dont les groupes capables d'établir des interactions hydrogènes sont disposés dans la chaîne du polymère.
Les polymères siliconés peuvent plus particulièrement être des polymères comprenant au moins un motif répondant

à la formule générale I :

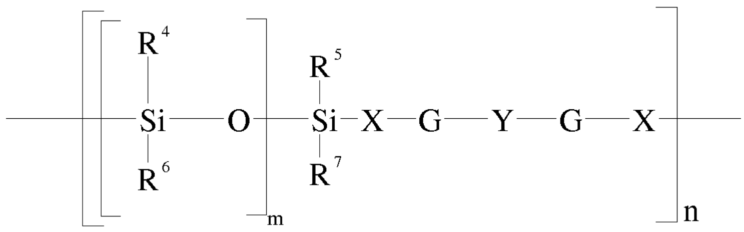

(I)

dans laquelle :

1) $R^4$, $R^5$, $R^6$ et $R^7$, identiques ou différents, représentent un groupe choisi parmi:

- les groupes hydrocarbonés, linéaires, ramifiés ou cycliques, en $C_1$ à $C_{40}$, saturés ou insaturés, pouvant contenir dans leur chaîne un ou plusieurs atomes d'oxygène, de soufre et/ou d'azote, et pouvant être substitués en partie ou totalement par des atomes de fluor,
- les groupes aryles en $C_6$ à $C_{10}$, éventuellement substitués par un ou plusieurs groupes alkyle en $C_1$ à $C_4$,
- les chaînes polyorganosiloxanes contenant ou non un ou plusieurs atomes d'oxygène, de soufre et/ou d'azote,

2) les X, identiques ou différents, représentent un groupe alkylène di-yle, linéaire ou ramifié en $C_1$ à $C_{30}$, pouvant contenir dans sa chaîne un ou plusieurs atomes d'oxygène et/ou d'azote,
3) Y est un groupe divalent alkylène linéaire ou ramifié, arylène, cycloalkylène, alkylarylène ou arylalkylène, saturé ou insaturé, en $C_1$ à $C_{50}$, pouvant comporter un ou plusieurs atomes d'oxygène, de soufre et/ou d'azote, et/ou porter comme substituant l'un des atomes ou groupes d'atomes suivants : fluor, hydroxy, cycloalkyle en $C_3$ à $C_8$, alkyle en $C_1$ à $C_{40}$, aryle en $C_5$ à $C_{10}$, phényle éventuellement substitué par 1 à 3 groupes alkyle en $C_1$ à $C_3$, hydroxyalkyle en $C_1$ à $C_3$ et amino alkyle en $C_1$ à $C_6$, ou
4) Y représente un groupe répondant à la formule :

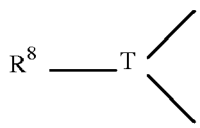

dans laquelle

- T représente un groupe hydrocarboné trivalent ou tétravalent, linéaire ou ramifié, saturé ou insaturé, en $C_3$ à $C_{24}$ éventuellement substitué par une chaîne polyorganosiloxane, et pouvant contenir un ou plusieurs atomes choisis parmi O, N et S, ou T représente un atome trivalent choisi parmi N, P et Al, et
- $R^8$ représente un groupe alkyle en $C_1$ à $C_{50}$, linéaire ou ramifié, ou une chaîne polyorganosiloxane, pouvant comporter un ou plusieurs groupes ester, amide, uréthane, thiocarbamate, urée, thiourée et/ou sulfonamide qui peut être lié ou non à une autre chaîne du polymère,

5) les G, identiques ou différents, représentent les groupes divalents choisis parmi :

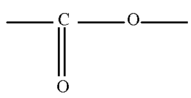 ; 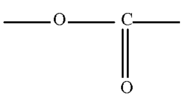 ; 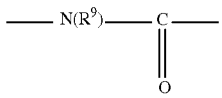 ;

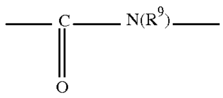 ; 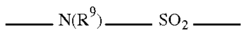 ; 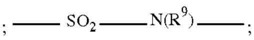 ;

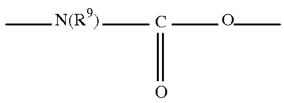 ; 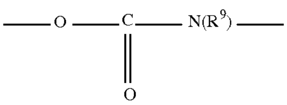 ; 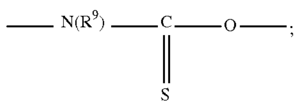 ;

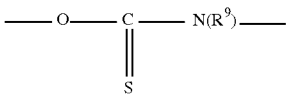 ; 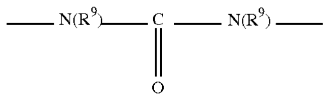 ,

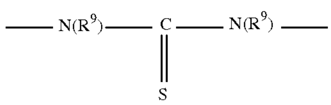 ,

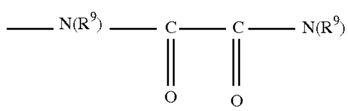 ; 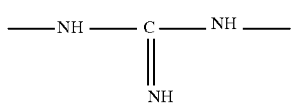 ; et

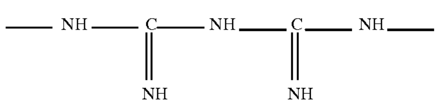

où $R^9$ représente un atome d'hydrogène ou un groupe alkyle, linéaire ou ramifié, en $C_1$ à $C_{20}$, à condition qu'au moins 50 % des $R^9$ du polymère représente un atome d'hydrogène et qu'au moins deux des groupes G du polymère soient un autre groupe que :

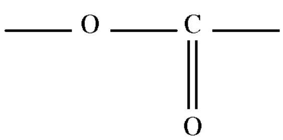 et 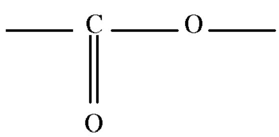 ;

6) n est un nombre entier allant de 2 à 500, de préférence de 2 à 200, et m est un nombre entier allant de 1 à

1000, de préférence de 1 à 700 et mieux encore de 6 à 200.

Selon l'invention, 80 % des $R^4$, $R^5$, $R^6$ et $R^7$, du polymère sont choisis de préférence parmi les groupes méthyle, éthyle, phényle et 3,3,3-trifluoropropyle.

Selon l'invention, Y peut représenter divers groupes divalents, comportant éventuellement de plus une ou deux valences libres pour établir des liaisons avec d'autres motifs du polymère ou copolymère. De préférence, Y représente un groupe choisi parmi :

a) les groupes alkylène linéaires en $C_1$ à $C_{20}$, de préférence en $C_1$ à $C_{10}$,
b) les groupes alkylène ramifiés pouvant comporter des cycles et des insaturations non conjuguées, en $C_{30}$ à $C_{56}$,
c) les groupes cycloalkylène en $C_5$-$C_6$,
d) les groupes phénylène éventuellement substitués par un ou plusieurs groupes alkyle en $C_1$ à $C_{40}$,
e) les groupes alkylène en $C_1$ à $C_{20}$, comportant de 1 à 5 groupes amides,
f) les groupes alkylène en $C_1$ à $C_{20}$, comportant un ou plusieurs substituants, choisis parmi les groupes hydroxyle, cycloalcane en $C_3$ à $C_8$, hydroxyalkyle en $C_1$ à $C_3$ et alkylamines en $C_1$ à $C_6$,
g) les chaînes polyorganosiloxane de formule :

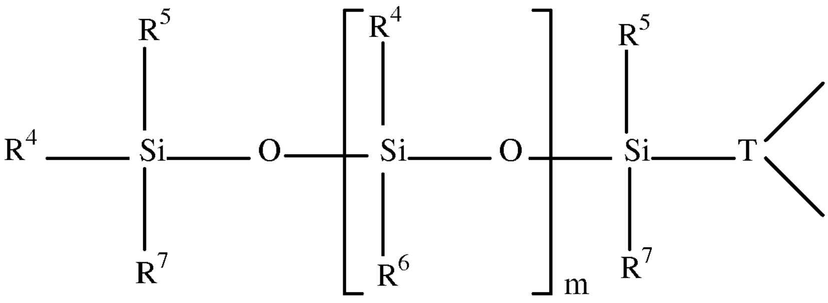

dans laquelle $R^4$, $R^5$, $R^6$, $R^7$, T et m sont tels que définis ci-dessus, et
h) les chaînes polyorganosiloxanes de formule :

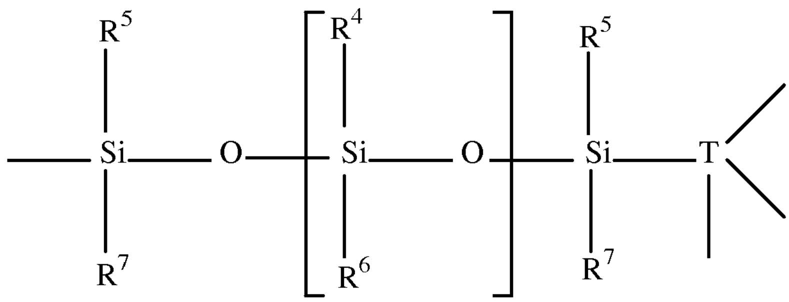

b) Selon la seconde variante, les polyorganosiloxanes peuvent être des polymères comprenant au moins un motif répondant à la formule (II) :

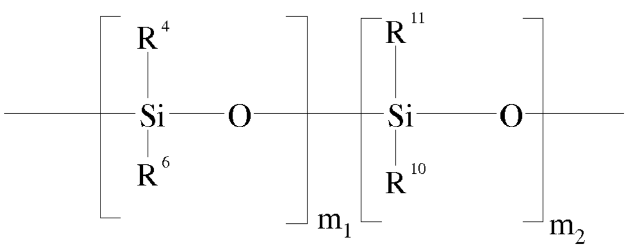

(II)

dans laquelle

- $R^4$ et $R^6$, identiques ou différents, sont tels que définis ci-dessus pour la formule (I),
- $R^{10}$ représente un groupe tel que défini ci-dessus pour $R^4$ et $R^6$, ou représente le groupe de formule -X-G-$R^{12}$ dans laquelle X et G sont tels que définis ci-dessus pour la formule (I) et $R^{12}$ représente un atome d'hydrogène ou un groupe hydrocarboné, linéaire, ramifié ou cyclique, saturé ou insaturé, en $C_1$ à $C_{50}$ comportant éventuellement dans sa chaîne un ou plusieurs atomes choisis parmi O, S et N, éventuellement substitué par un

ou plusieurs atomes de fluor et/ou un ou plusieurs groupes hydroxyle, ou un groupe phényle éventuellement substitué par un ou plusieurs groupes alkyle en $C_1$ à $C_4$,

- $R^{11}$ représente le groupe de formule -X-G-$R^{12}$ dans laquelle X, G et $R^{12}$ sont tels que définis ci-dessus,
- $m_1$ est un nombre entier allant de 1 à 998, et
- $m_2$ est un nombre entier allant de 2 à 500.

Selon l'invention, le polymère utilisé comme agent structurant, peut être un homopolymère, c'est-à-dire un polymère comportant plusieurs motifs identiques, en particulier des motifs de formule (I) ou de formule (II).

Selon l'invention, on peut aussi utiliser un polymère constitué par un copolymère comportant plusieurs motifs de formule (I) différents, c'est-à-dire un polymère dans lequel l'un au moins des $R^4$, $R^5$, $R^6$, $R^7$, X, G, Y, m et n est différent dans l'un des motifs. Le copolymère peut être aussi formé de plusieurs motifs de formule (II), dans lequel l'un au moins des $R^4$, $R^6$, $R^{10}$, $R^{11}$, m1 et m2 est différent dans l'un au moins des motifs.

On peut encore utiliser un polymère comportant au moins un motif de formule (I) et au moins un motif de formule (II), les motifs de formule (I) et les motifs de formule (II) pouvant être identiques ou différents les uns des autres.

Selon une variante de l'invention, on peut encore utiliser un polymère comprenant de plus au moins un motif hydrocarboné comportant deux groupes capables d'établir des interactions hydrogènes choisis parmi les groupes ester, amide, sulfonamide, carbamate, thiocarbamate, urée, uréthane, thiourée, oxamido, guanidino, biguanidino et leurs combinaisons.

Ces copolymères peuvent être des polymères blocs, des polymères séquencés ou des polymères greffés.

Selon un mode de réalisation avantageux de l'invention, les groupes capables d'établir des interactions hydrogènes sont des groupes amides de formule -C(O)NH- et -HN-C(O)-.

Dans ce cas, l'agent structurant peut être un polymère comprenant au moins un motif de formule (III) ou (IV) :

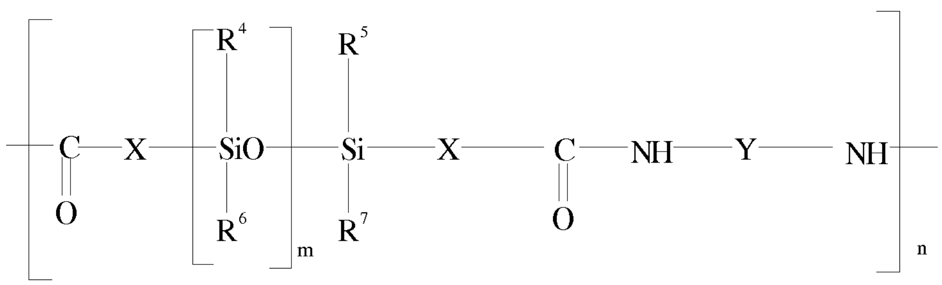

(III)

ou

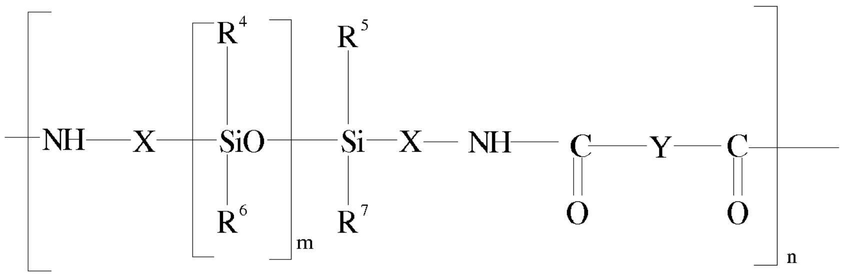

(IV)

dans lesquelles $R^4$, $R^5$, $R^6$, $R^7$, X, Y, m et n sont tels que définis ci-dessus.

Un tel motif peut être obtenu :

- soit par une réaction de condensation entre un silicone à extrémités α, ω-acides carboxyliques et une ou plusieurs diamines, selon le schéma réactionnel suivant :

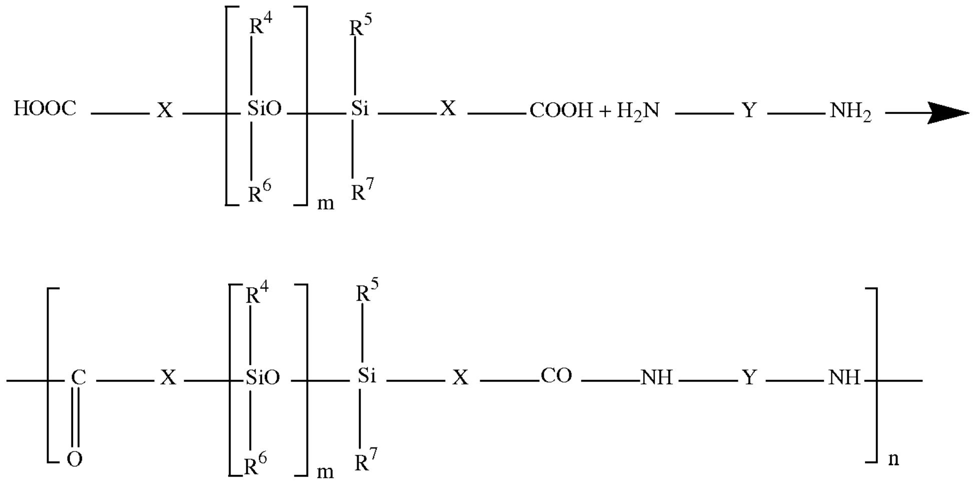

- soit par réaction de deux molécules d'acide carboxylique α-insaturé avec une diamine selon le schéma réactionnel suivant :

$$CH_2{=}CH\text{-}X^1\text{-}COOH{+}H_2N\text{-}Y\text{-}NH_2 \rightarrow CH_2{=}CH\text{-}X^1\text{-}CO\text{-}NH\text{-}Y\text{-}NH\text{-}CO\text{-}X^1\text{-}CH{=}CH_2$$

suivie de l'addition d'un siloxane sur les insaturations éthyléniques, selon le schéma suivant : $CH_2{=}CH\text{-}X^1\text{-}CO\text{-}NH\text{-}Y\text{-}NH\text{-}CO\text{-}X^1\text{-}CH{=}CH_2$

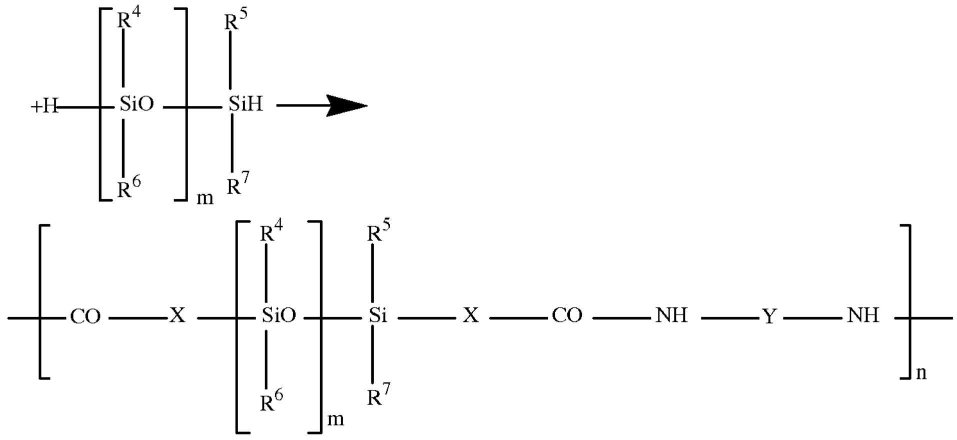

dans lesquels $X^1$-$(CH_2)_2$- correspond au X défini ci-dessus et Y, $R^4$, $R^5$, $R^6$, $R^7$ et m sont tels que définis ci-dessus,

- soit par réaction d'un silicone à extrémités α, ω-$NH_2$ et d'un diacide de formule HOOC-Y-COOH selon le schéma réactionnel suivant :

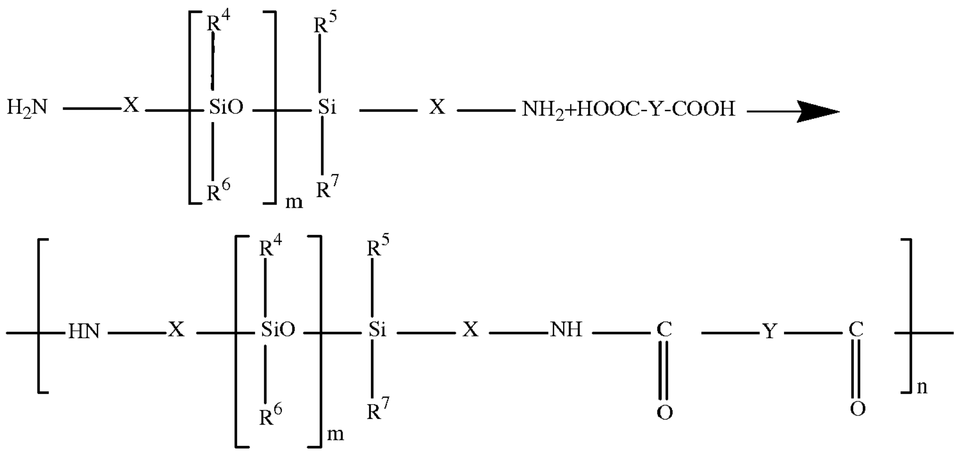

Dans ces polyamides de formule (III) ou (IV), m va de 1 à 700, en particulier de 15 à 500 et notamment de 50 à 200 et n va particulier de 1 à 500, de préférence de 1 à 100 et mieux encore de 4 à 25,

- X est de préférence une chaîne alkylène linéaire ou ramifiée ayant de 1 à 30 atomes de carbone, en particulier 1 à 20 atomes de carbone, notamment de 5 à 15 atomes de carbone et plus particulièrement de 10 atomes de carbone, et
- Y est de préférence une chaîne alkylène linéaire ou ramifiée ou pouvant comporter des cycles et/ou des insaturations, ayant de 1 à 40 atomes de carbone, en particulier de 1 à 20 atomes de carbone, et mieux encore de 2 à 6 atomes de carbone, en particulier de 6 atomes de carbone.

Dans les formules (III) et (IV), le groupe alkylène représentant X ou Y peut éventuellement contenir dans sa partie alkylène au moins l'un des éléments suivants :

1) 1 à 5 groupes amides, urée, uréthane, ou carbamate,
2) un groupe cycloalkyle en $C_5$ ou $C_6$, et
3) un groupe phénylène éventuellement substitué par 1 à 3 groupes alkyles identiques ou différents en $C_1$ à $C_3$.

Dans les formules (III) et (IV), les groupes alkylènes peuvent aussi être substitués par au moins un élément choisi dans le groupe constitué de :

- un groupe hydroxy,

- un groupe cycloalkyle en $C_3$ à $C_8$,
- un à trois groupes alkyles en $C_1$ à $C_{40}$,
- un groupe phényle éventuellement substitué par un à trois groupes alkyles en $C_1$ à $C_3$,
- un groupe hydroxyalkyle en $C_1$ à $C_3$, et
- un groupe aminoalkyle en $C_1$ à $C_6$.

Dans ces formules (III) et (IV), Y peut aussi représenter :

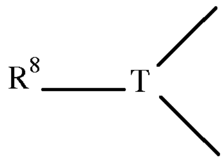

où $R^8$ représente une chaîne polyorganosiloxane, et T représente un groupe de formule :

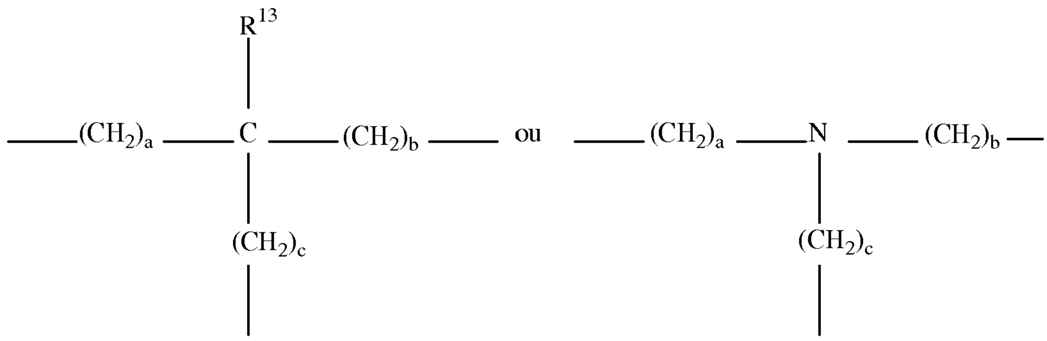

dans lesquelles a, b et c sont indépendamment des nombres entiers allant de 1 à 10, et $R^{13}$ est un atome d'hydrogène ou un groupe tel que ceux définis pour $R^4$, $R^5$, $R^6$ et $R^7$.

Dans les formules (III) et (IV), $R^4$, $R^5$, $R^6$ et $R^7$ représentent de préférence, indépendamment, un groupe alkyle en $C_1$ à $C_{40}$, linéaire ou ramifié, de préférence un groupe $CH_3$, $C_2H_5$, n-$C_3H_7$ ou isopropyle, une chaîne polyorganosiloxane ou un groupe phényle éventuellement substitué par un à trois groupes méthyle ou éthyle.

Comme on l'a vu précédemment, le polymère peut comprendre des motifs de formule (III) ou (IV) identiques ou différents.

Ainsi, le polymère peut être un polyamide contenant plusieurs motifs de formule (III) ou (IV) de longueurs différentes, soit un polyamide répondant à la formule (V) :

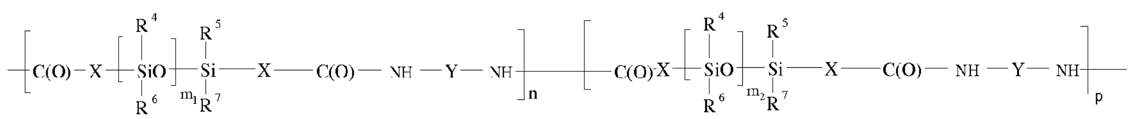

(V)

dans laquelle X, Y, n, $R^4$ à $R^7$ ont les significations données ci-dessus, $m_1$ et $m_2$ qui sont différents, sont choisis dans la gamme allant de 1 à 1000, et p est un nombre entier allant de 2 à 300.

Dans cette formule, les motifs peuvent être structurés pour former soit un copolymère bloc, soit un copolymère aléatoire, soit un copolymère alterné. Dans ce copolymère, les motifs peuvent être non seulement de longueurs différentes mais aussi de structures chimiques différentes, par exemple ayant des Y différents. Dans ce cas, le polymère peut répondre à la formule VI:

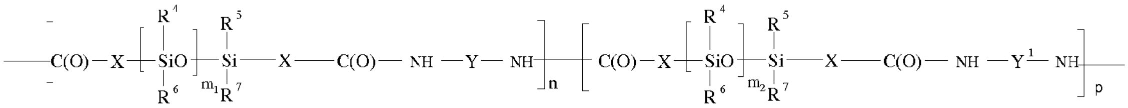

(VI)

dans laquelle $R^4$ à $R^7$, X, Y, $m_1$, $m_2$, n et p ont les significations données ci-dessus et $Y^1$ est différent de Y mais choisi parmi les groupes définis pour Y. Comme précédemment, les différents motifs peuvent être structurés pour former soit un copolymère bloc, soit un copolymère aléatoire, soit un copolymère alterné.

Dans ce premier mode de réalisation de l'invention, l'agent structurant peut être aussi constitué par un copolymère greffé. Ainsi, le polyamide à unités silicone peut être greffé et éventuellement réticulé par des chaînes silicones à groupes amides. De tels polymères peuvent être synthétisés avec des amines trifonctionnelles.

Dans ce cas, le polymère peut comprendre au moins un motif de formule (VII) :

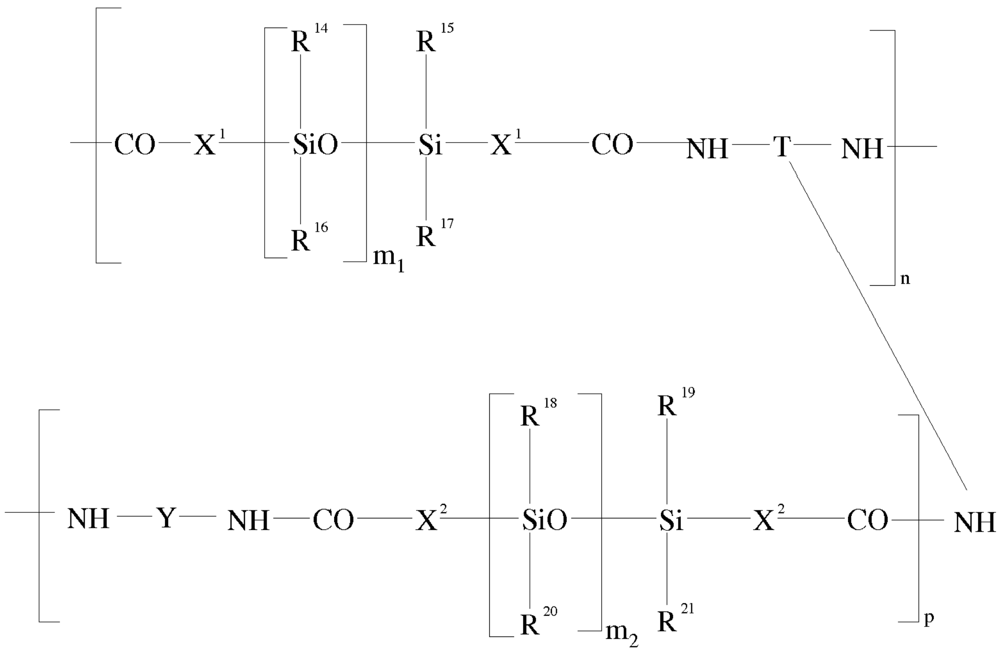

(VII)

dans laquelle $X^1$ et $X^2$ qui sont identiques ou différents, ont la signification donnée pour X dans la formule (I), n est tel que défini dans la formule (I), Y et T sont tels que définis dans la formule (I), $R^{14}$ à $R^{21}$ sont des groupes choisis dans le même groupe que les $R^4$ à $R^7$, $m_1$ et $m_2$ sont des nombres situés dans la gamme allant de 1 à 1 000, et p est un nombre entier allant de 2 à 500.

Dans la formule (VII), on préfère que :

- p soit va de 1 à 25, mieux encore de 1 à 7,
- $R^{14}$ à $R^{21}$ soient des groupes méthyle,
- T réponde à l'une des formules suivantes :

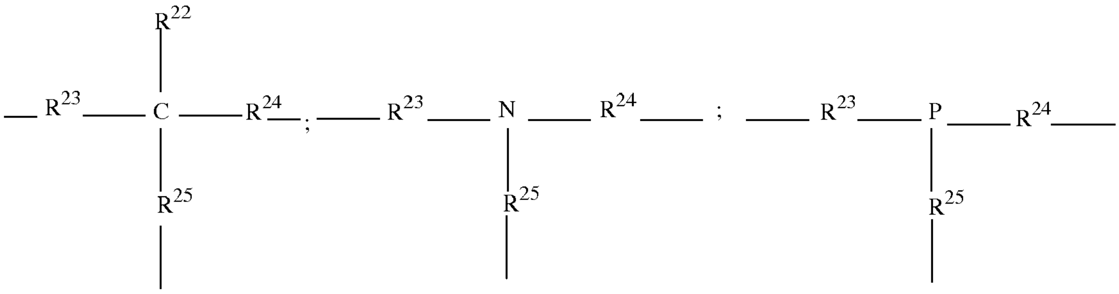

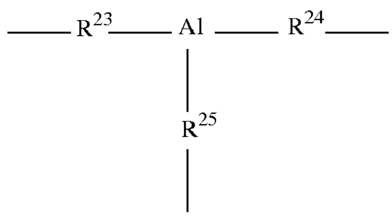

dans lesquelles $R^{22}$ est un atome d'hydrogène ou un groupe choisi parmi les groupes définis pour $R^4$ à $R^7$, et $R^{23}$, $R^{24}$ et $R^{25}$ sont indépendamment des groupes alkylène, linéaires ou ramifiés, de préférence encore, à la formule :

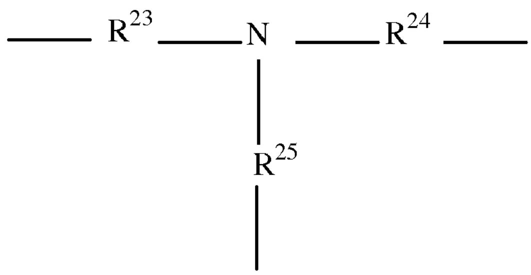

en particulier avec $R^{23}$, $R^{24}$ et $R^{25}$ représentant $-CH_2-CH_2-$,

- $m_1$ et $m_2$ vont de 15 à 500, et mieux encore de 15 à 45,
- $X_1$ et $X_2$ représentent $-(CH_2)_{10}-$, et
- Y représente $-CH_2-$.

Ces polyamides à motif silicone greffé de formule (VII) peuvent être copolymérisés avec des polyamides-silicones de formule (II) pour former des copolymères blocs, des copolymères alternés ou des copolymères aléatoires. Le pourcentage en poids de motifs silicone greffé (VII) dans le copolymère peut aller de 0,5 à 30 % en poids.

Selon l'invention, comme on l'a vu précédemment, les unités siloxanes peuvent être dans la chaîne principale ou squelette du polymère, mais elles peuvent également être présentes dans des chaînes greffées ou pendantes. Dans la chaîne principale, les unités siloxanes peuvent être sous forme de segments comme décrits ci-dessus. Dans les chaînes pendantes ou greffées, les unités siloxanes peuvent apparaître individuellement ou en segments.

Selon une variante de réalisation de l'invention, on peut utiliser un copolymère de polyamide silicone et de polyamide hydrocarboné, soit un copolymère comportant des motifs de formule (III) ou (IV) et des motifs polyamide hydrocarboné. Dans ce cas, les motifs polyamide-silicone peuvent être disposés aux extrémités du polyamide hydrocarboné.

Selon un mode de réalisation particulier, la composition comprend au moins un polymère bloc polydiméthylsiloxane de formule générale (I) possédant un indice m de valeur environ 100. De préférence encore, la composition selon l'invention comprend au moins un polymère comprenant au moins un motif de formule (III) où m varie de 50 à 600, en particulier de 60 à 400, notamment de 75 à 200 et plus particulièrement est de l'ordre de 100 et de préférence encore, $R^4$, $R^5$, $R^6$ et $R^7$ représentent indépendamment, dans la formule (III), un groupe alkyle en $C_1$ à $C_{40}$, linéaire ou

ramifié, de préférence un groupe $CH_3$, $C_2H_5$, n-$C_3H_7$ ou isopropyle.

De préférence encore, X et Y représentent indépendemment un groupe choisi parmi les groupes alkylène linéaires en $C_1$ à $C_{20}$, de préférence en $C_1$ à $C_{10}$.

A titre d'exemples de polymère siliconé utilisable, on peut citer un des polyamides siliconés, obtenus conformément aux exemples 1 à 3 du document US-A-5 981 680.

Selon une variante de réalisation de l'invention, le polymère est constitué par un homopolymère ou copolymère comportant des groupes uréthane ou urée. Ces polymères sont décrits en détail dans la demande WO 2003/106614 publiée le 24/12/2003 .

Comme précédemment, un tel polymère peut comporter des motifs polyorganosiloxanes contenant deux ou plusieurs groupes uréthanes et/ou urées, soit dans le squelette du polymère, soit sur des chaînes latérales ou comme groupes pendants.

Les polymères comportant au moins deux groupes uréthanes et/ou urées dans le squelette peuvent être des polymères comprenant au moins un motif répondant à la formule suivante (VIII) :

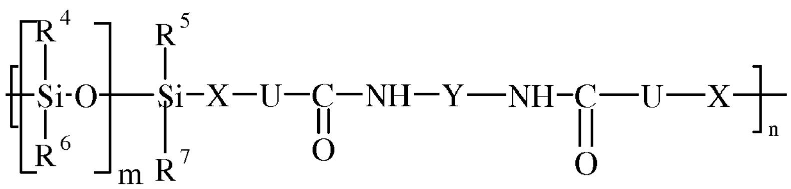

(VIII)

dans laquelle les $R^4$, $R^5$, $R^6$, $R^7$, X, Y, m et n ont les significations données ci-dessus pour la formule (I), et U représente -O- ou -NH-, afin que :

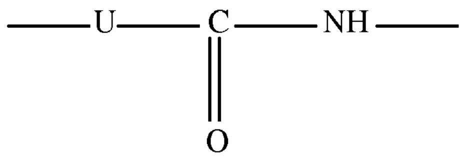

corresponde à un groupe uréthane ou urée.

Dans cette formule (VIII), Y peut être un groupe alkylène, en $C_1$ à $C_{40}$, linéaire ou ramifié, substitué éventuellement par un groupe alkyle en $C_1$ à $C_{15}$ ou un groupe aryle en $C_5$ à $C_{10}$. De préférence, on utilise un groupe $-(CH_2)_6-$.

Y peut aussi représenter un groupe cycloaliphatique ou aromatique en $C_5$ à $C_{12}$ pouvant être substitué par un groupe alkyle en $C_1$ à $C_{15}$ ou un groupe aryle en $C_5$ à $C_{10}$, par exemple un radical choisi parmi le radical méthylène-4-4-biscyclohexyle, le radical dérivé de l'isophorone diisocyanate, les 2,4 et 2,6-tolylènes, le 1,5-naphtylène, le p-phénylène et le 4,4'-biphénylène méthane. Généralement, on préfère que Y représente un radical alkylène en $C_1$ à $C_{40}$, linéaire ou ramifié, ou un radical cycloalkylène en $C_4$ à $C_{12}$.

Y peut aussi représenter une séquence polyuréthane ou polyurée correspondant à la condensation de plusieurs molécules de diisocyanate avec une ou plusieurs molécules de coupleurs du type diol ou diamine. Dans ce cas, Y comprend plusieurs groupes uréthane

ou urée dans la chaîne alkylène.

Il peut répondre à la formule (IX) :

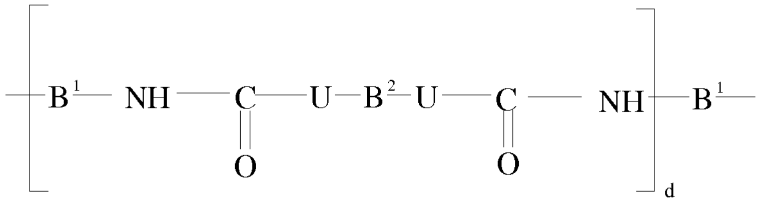

(IX)

dans laquelle $B^1$ est un groupe choisi parmi les groupes donnés ci-dessus pour Y, U est -O- ou -NH-, et $B^2$ est choisi parmi :

- les groupes alkylène en $C_1$ à $C_{40}$, linéaires ou ramifiés,
- les groupes cycloalkylène en $C_5$ à $C_{12}$, éventuellement porteurs de substituants alkyle, par exemple un à trois groupes méthyle ou éthyle, ou alkylène, par exemple le radical du diol : cyclohexane diméthanol,
- les groupes phénylène pouvant éventuellement être porteurs de substituants alkyles en $C_1$ à $C_3$, et
- les groupes de formule :

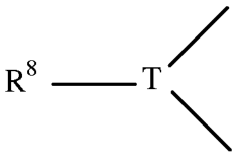

dans laquelle T est un radical trivalent hydrocarboné pouvant contenir un ou plusieurs hétéroatomes tels que l'oxygène, le soufre et l'azote et $R^8$ est une chaîne polyorganosiloxane
ou une chaîne alkyle en $C_1$ à $C_{50}$, linéaire ou ramifiée.

T peut représenter par exemple :

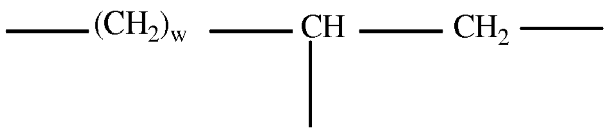

ou

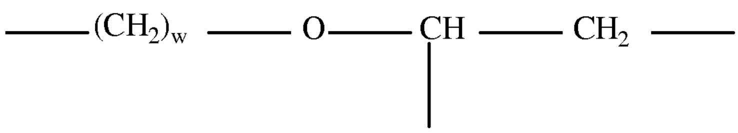

avec w étant un nombre entier allant de 1 à 10 et $R^8$ étant une chaîne polyorganosiloxane.
Lorsque Y est un groupe alkylène, en $C_1$ en $C_{40}$ linéaire ou ramifié, on préfère les groupes $-(CH_2)_2-$ et $-(CH_2)_6-$.
Dans la formule donnée ci-dessus pour Y, d peut être un entier allant de 0 à 5, de préférence de 0 à 3, de préférence encore égal à 1 ou 2.
De préférence $B^2$ est un groupe alkylène en $C_1$ à $C_{40}$, linéaire ou ramifié, en particulier $-(CH_2)_2-$ ou $-(CH_2)_6-$, ou le groupe :

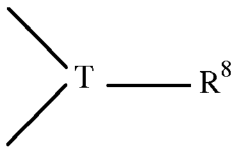

avec $R^8$ étant une chaîne polyorganosiloxane.

Comme précédemment, le polymère siliconé peut être formé de motifs silicone uréthane et/ou silicone-urée de longueur et/ou de constitution différentes, et se présenter sous la forme de copolymères blocs, séquencés ou statistiques (aléatoires).

Les polymères de formule (VIII) comportant des groupes urées ou uréthanes dans la chaîne du polymère siliconé peuvent être obtenus par réaction entre un silicone à groupes terminaux $\alpha,\omega$-$NH_2$ ou -OH, de formule :

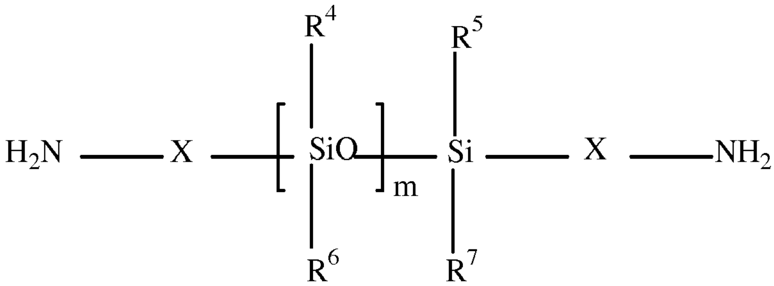

dans laquelle m, $R^4$, $R^5$, $R^6$, $R^7$ et X sont tels que définis pour la formule (I), et un diisocyanate OCN-Y-NCO où Y a la signification donnée dans la formule (I); et éventuellement un coupleur diol ou diamine de formule $H_2N$-$B^2$-$NH_2$ ou HO-$B^2$-OH, où $B^2$ est tel que défini dans la formule (IX).

Suivant les proportions stoechiométriques entre les deux réactifs, diisocyanate et coupleur, on pourra avoir pour Y la formule (IX) avec d égale 0 où d égale 1 à 5.

Comme dans le cas des polyamides silicones de formule (IV), (II) ou (III), on peut utiliser dans l'invention des polyuréthanes ou des polyurées silicones ayant des motifs de longueur et de structure différentes, en particulier des motifs de longueurs différentes par le nombre d'unités silicones. Dans ce cas, le copolymère peut répondre par exemple à la formule :

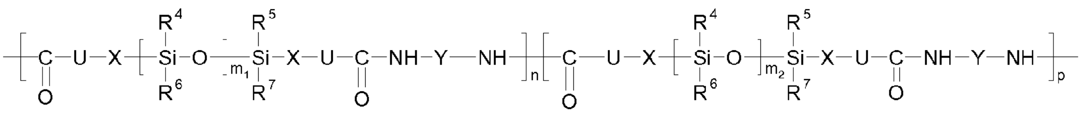

(XII)

dans laquelle $R^4$, $R^5$, $R^6$, $R^7$, X, Y et U sont tels que définis pour la formule (VIII) et $m_1$, $m_2$, n et p sont tels que définis pour la formule (V).

Selon l'invention, le silicone peut aussi comporter les groupes uréthane et/ou urée non plus dans le squelette mais en ramifications latérales.

Dans ce cas, le polymère peut comprendre au moins un motif de formule :

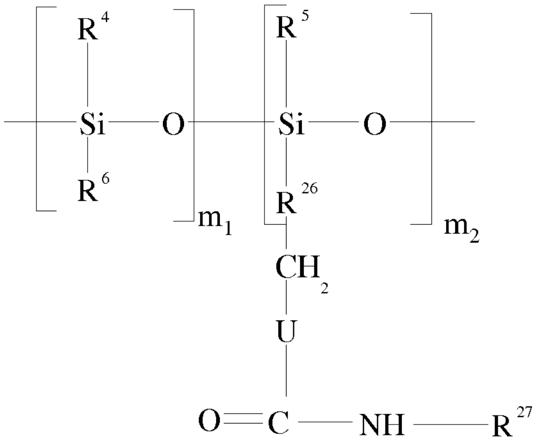

(X)

dans laquelle $R^4$, $R^6$, $R^5$, $m_1$ et $m_2$ ont les significations données ci-dessus pour la formule (II), et $R^5$ pour la formule (I),

- U représente O ou NH,
- $R^{26}$ représente un groupe alkylène en $C_1$ à $C_{40}$, comportant éventuellement un ou plusieurs hétéroatomes choisis parmi O et N, ou un groupe phénylène, et
- $R^{27}$ est choisi parmi les groupes alkyle en $C_1$ à $C_{50}$, linéaires, ramifiés ou cycliques, saturés ou insaturés, et les groupes phényle éventuellement substitués par un à trois groupes alkyles en $C_1$ à $C_3$.

Les polymères comportant au moins un motif de formule (X) contiennent des unités siloxanes et des groupes urées ou uréthanes, et ils peuvent être utilisés comme polymère structurant dans les compositions de l'invention.
Les polymères siloxanes peuvent avoir un seul groupe urée ou uréthane par ramification ou peuvent avoir des ramifications à deux groupes urée ou uréthane, ou encore contenir un mélange de ramifications à un groupe urée ou uréthane et de ramifications à deux groupes urée ou uréthane.
Ils peuvent être obtenus à partir de polysiloxanes ramifiés, comportant un ou deux groupes amino par ramification, en faisant réagir ces polysiloxanes avec des monoisocyanates.
A titre d'exemples de polymères de départ de ce type ayant des ramifications amino et diamino, on peut citer les polymères répondant aux formules suivantes :

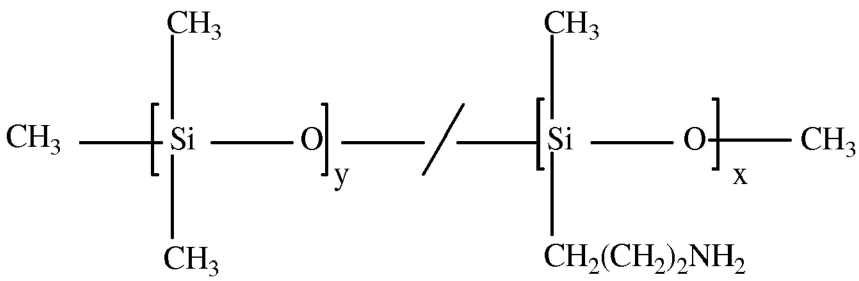

y=57 ; x = 3

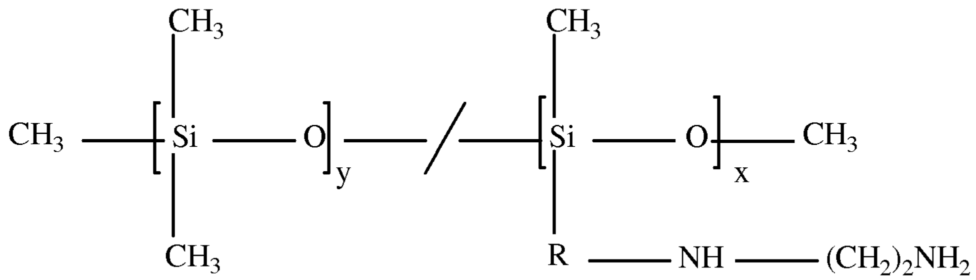

y=56 ; x = 4

Dans ces formules, le symbole "/" indique que les segments peuvent être de longueurs différentes et dans un ordre aléatoire, et R représente un groupe aliphatique linéaire ayant de préférence 1 à 6 atomes de carbone et mieux encore 1 à 3 atomes de carbone.
De tels polymères à ramification peuvent être formés en faisant réagir un polymère siloxane, ayant au moins trois groupes amino par molécule de polymère, avec un composé ayant un seul groupe monofonctionnel (par exemple un acide, un isocyanate ou isothiocyanate) pour faire réagir ce groupe monofonctionnel avec l'un des groupes amino et former les groupes capables d'établir des interactions hydrogène. Les groupes amino peuvent être sur des chaînes latérales s'étendant de la chaîne principale du polymère siloxane de sorte que les groupes capables d'établir des interactions hydrogène sont formés sur ces chaînes latérales,
ou bien les groupes amino peuvent être aux extrémités de la chaîne principale de sorte que les groupes capables d'interaction hydrogène seront des groupes terminaux du polymère.
Comme mode opératoire pour former un polymère contenant des unités siloxanes et des groupes capables d'établir des interactions hydrogène, on peut citer la réaction d'une siloxane diamine et d'un diisocyanate dans un solvant siliconé de façon à fournir directement un gel. La réaction peut être exécutée dans un fluide siliconé, le produit résultant étant dissous dans le fluide siliconé, à température élevée, la température du système étant ensuite diminuée pour former le gel.
Les polymères préférés pour l'incorporation dans les compositions selon la présente invention, sont des copolymères siloxanes-urées qui sont linéaires et qui contiennent des groupes urées comme groupes capables d'établir des interactions hydrogène dans le squelette du polymère.
A titre d'illustration d'un polysiloxane terminé par quatre groupes urées, on peut citer le polymère de formule :

(Ph = Phényle)

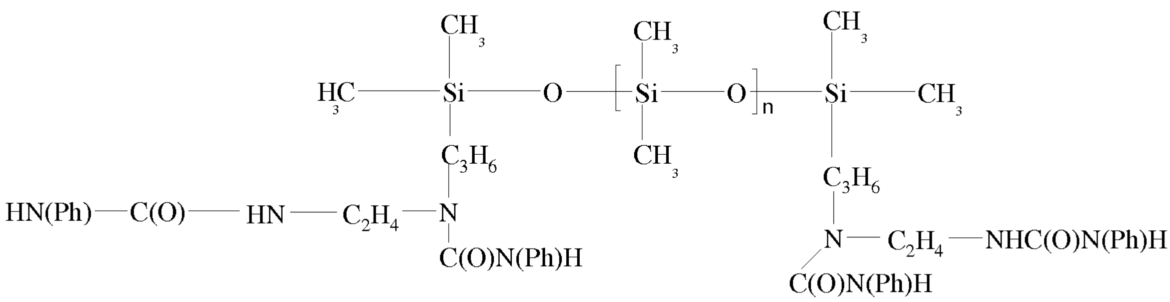

(XI)

où Ph est un groupe phényle et n est un nombre de 0 à 300, en particulier de 0 à 100, par exemple de 50.
Ce polymère est obtenu par réaction du polysiloxane à groupes amino suivant :

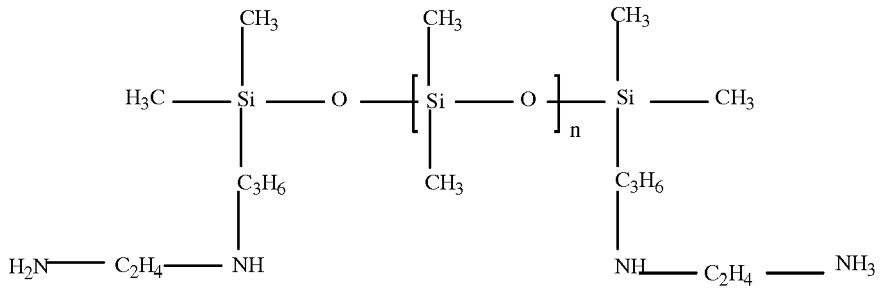

(n-50)

avec l'isocyanate de phényle.

On peut obtenir également des polyuréthanes ou polyurées silicones ramifiés en utilisant à la place du diisocyanate OCN-Y-NCO, un triisocyanate de formule :

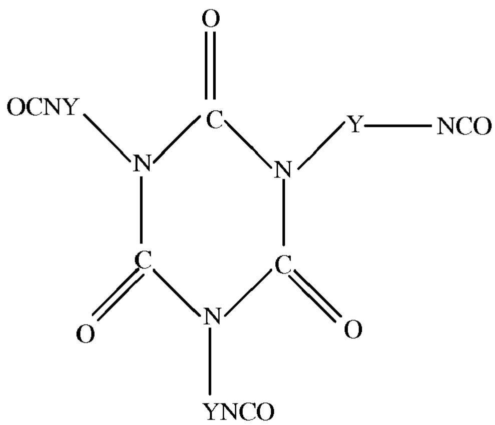

On obtient ainsi une polyuréthane ou polyurée silicone ayant des ramifications comportant une chaîne organosiloxane avec des groupes capables d'établir des interactions hydrogène. Un tel polymère comprend par exemple un motif répondant à la formule :

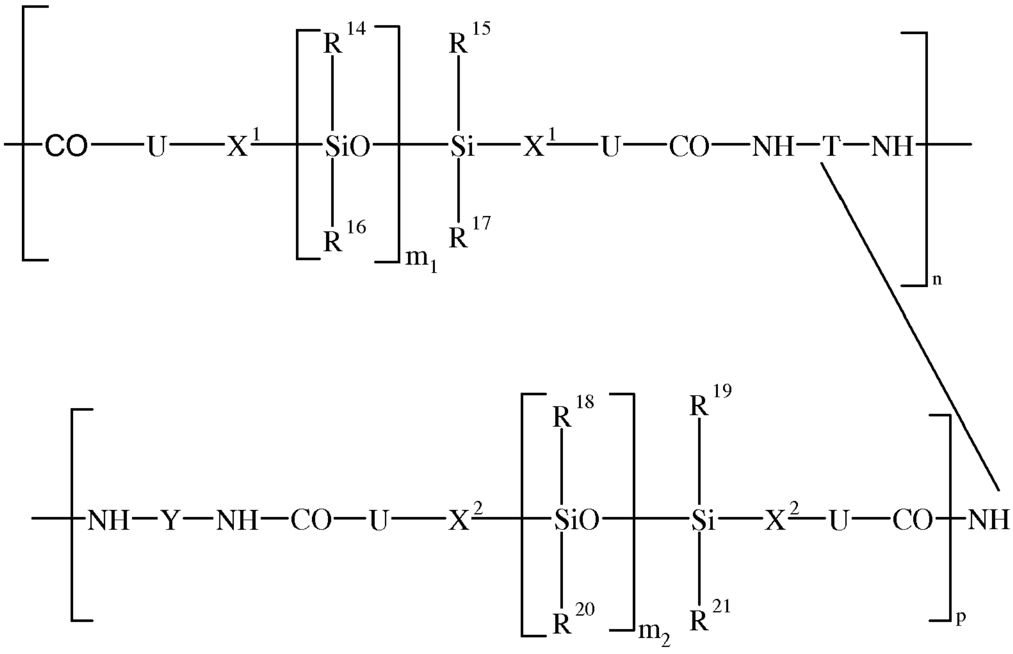

(XIII)

dans laquelle $X^1$ et $X^2$ qui sont identiques ou différents, ont la signification donnée pour X dans la formule (I), n est tel que défini dans la formule (I), Y et T sont tels que définis dans la formule (I), $R^{14}$ à $R^{21}$ sont des groupes choisis dans le même groupe que les $R^4$ à $R^7$, $m_1$ et $m_2$ sont des nombres situés dans la gamme allant de 1 à 1 000, et p est un nombre entier allant de 2 à 500.

Comme dans le cas des polyamides, on peut utiliser dans l'invention des copolymères de polyuréthane -ou de polyurée-silicone et de polyuréthane ou polyurée hydrocarboné en réalisant la réaction de synthèse du polymère en présence d'une séquence α, ω-difonctionnelle de nature non silicone, par exemple un polyester, un polyéther ou une polyoléfine.

Comme on l'a vu précédemment, les copolymères de l'invention peuvent avoir des motifs siloxanes dans la chaîne principale du polymère et des groupes capables d'établir des interactions hydrogène, soit dans la chaîne principale du polymère ou aux extrémités de celle-ci, soit sur des chaînes latérales ou ramifications de la chaîne principale. Ceci peut correspondre aux cinq dispositions suivantes :

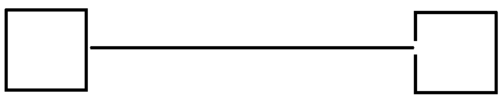

(1)

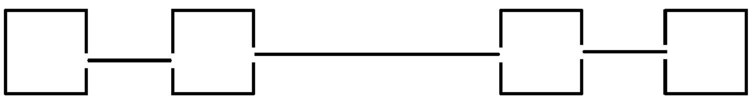

(2)

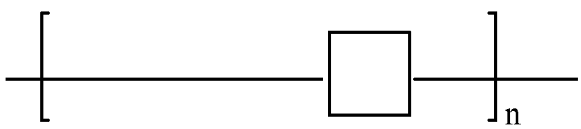

(3)

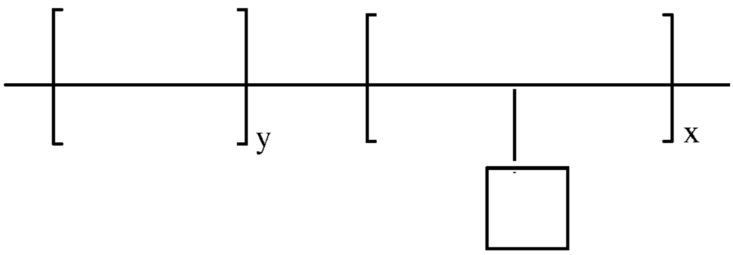

(4)

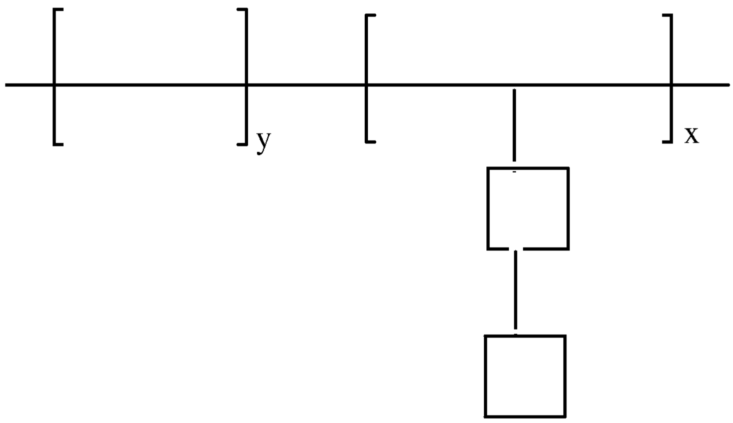

(5)

dans lesquelles, la ligne continue est la chaîne principale du polymère siloxane et les carrés représentent les groupes capables d'établir des interactions hydrogène.

Dans le cas (1), les groupes capables d'établir des interactions hydrogène sont disposés aux extrémités de la chaîne principale. Dans le cas (2), deux groupes capables d'établir des interactions hydrogène, sont disposés à chacune des extrémités de la chaîne principale.

Dans le cas (3), les groupes capables d'établir des interactions hydrogène sont disposés à l'intérieur de la chaîne principale dans des motifs répétitifs.

Dans les cas (4) et (5), il s'agit de copolymères dans lesquels les groupes capables d'établir des interactions hydrogène sont disposés sur des ramifications de la chaîne principale d'une première série de motifs qui sont copolymérisés avec des motifs ne comportant pas de groupes capables d'établir des interactions hydrogène.

Les polymères et copolymères utilisés dans la composition de l'invention ont avantageusement une température de transition solide-liquide de 45°C à 190°C. De préférence, ils présentent une température de transition solide-liquide allant de 70 à 130°C et mieux de 80°C à 105°C.

Ces polymères peuvent présenter une masse moléculaire moyenne en poids de 5000 à 500 000, par exemple de 10 000 à 300 000, de préférence de 20 000 à 150 000.

**<u>B/ Polymères polyamides</u>**

Comme exposé plus haut, le polymère polyamide comprend a) un squelette polymérique ayant des motifs de répétition hydrocarbonés pourvus d'au moins un motif amide non pendant, et éventuellement b) au moins une chaîne grasse pendante et/ou au moins une chaîne grasse terminale éventuellement fonctionnalisées, comprenant au moins 4 atomes de carbone et étant liées à ces motifs hydrocarbonés.

Par "chaînes fonctionnalisées" au sens de l'invention, on entend une chaîne alkyle comportant un ou plusieurs groupes fonctionnels ou réactifs notamment choisis parmi les groupes amides, hydroxyle, éther, oxyalkylène ou polyoxyalkylène, halogène, dont les groupes fluorés ou perfluorés, ester, siloxane, polysiloxane. En outre, les atomes d'hydrogène d'une ou plusieurs chaînes grasses peuvent être substituées au moins partiellement par des atomes de fluor.

Par "motifs de répétition hydrocarbonés", on entend au sens de l'invention un motif comportant de 2 à 80 atomes de carbone, et de préférence de 2 à 60 atomes de carbone, portant des atomes d'hydrogène et éventuellement des atomes d'oxygène, qui peut être linéaire, ramifié ou cyclique, saturé ou insaturé. Ces motifs comprennent, en outre, chacun au moins un groupement amide avantageusement non pendants et se trouvant dans le squelette polymérique.

Avantageusement, les chaînes pendantes sont liées directement à l'un au moins des atomes d'azote du squelette polymérique.

Le polyamide peut comprendre entre les motifs hydrocarbonés des motifs siliconés ou des motifs oxyalkylénés.

En outre, le polyamide de la composition de l'invention comprend avantageusement de 40 à 98 % de chaînes grasses par rapport au nombre total des motifs amide et des chaînes grasses et mieux de 50 à 95 %..

De préférence, les chaînes grasses pendantes sont liées à l'un au moins des atomes d'azote des motifs amide du polymère.
En particulier, les chaînes grasses de ce polyamide représentent de 40 à 98 % du nombre total des motifs amide et des chaînes grasses, et mieux de 50 à 95 %.

Avantageusement, le polyamide présente une masse moléculaire moyenne en poids inférieure à 100 000 (notamment allant de 1000 à 100 000), en particulier inférieure à 50 000 (notamment allant de 1000 à 50 000), et plus particulièrement allant de 1000 à 30 000, de préférence de 2000 à 20 000 ,et mieux de 2000 à 10 000.

Le polyamide est non soluble dans l'eau, notamment à 25 °C. En particulier, il ne comporte pas de groupe ionique.

Comme polyamides préférés utilisables dans l'invention, on peut citer les polyamides ramifiés par des chaînes grasses pendantes et/ou des chaînes grasses terminales ayant de 6 à 120 atomes de carbone et mieux de 8 à 120 et notamment de 12 à 68 atomes de carbone, chaque chaîne grasse terminale étant liée au squelette polyamide par au moins un groupe de liaison. Le groupe de liaison peut être choisi parmi les groupes ester, éther, amine, urée, uréthane, thioester, thioéther, thiurée, thiouréthane. De préférence, ces polymères comportent une chaîne grasse à chaque extrémité du squelette polyamide.

Ces polymères sont de préférence des polymères résultant d'une polycondensation entre un diacide carboxylique ayant au moins 32 atomes de carbone (ayant notamment de 32 à 44 atomes de carbone) avec une amine choisie parmi les diamines ayant au moins 2 atomes de carbone (notamment de 2 à 36 atomes de carbone) et les triamines ayant au moins 2 atomes de carbone (notamment de 2 à 36 atomes de carbone. Le diacide est de préférence un dimère issu d'acide gras à insaturation éthylénique ayant au moins 16 atomes de carbone, de préférence de 16 à 24 atomes de carbone, comme l'acide oléique, linoléique ou linolénique. La diamine est de préférence l'éthylène diamine, l'hexylène diamine, l'hexaméthylène diamine. La triamine est par exemple l'éthylène triamine. Pour les polymères comportant un ou 2 groupements d'acide carboxylique terminaux, il est avantageux de les estérifier par un monoalcool ayant au moins 4 atomes de carbone, de préférence de 10 à 36 atomes de carbone et mieux de 12 à 24 et encore mieux de 16 à 24, par exemple 18 atomes de carbone.

Le polyamide de la composition selon l'invention peut être en particulier choisi parmi les polymères de formule (A) suivante :

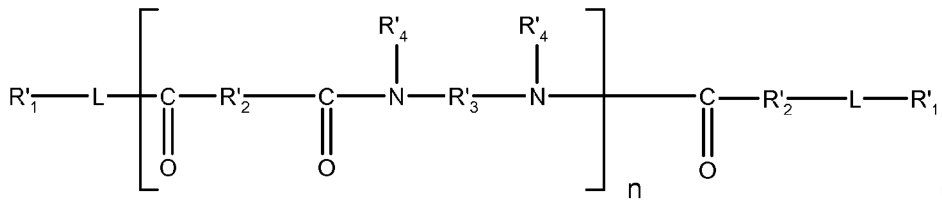

(A)

dans laquelle :

- n est un nombre entier allant de1 à 30,
- $R'^1$ représente à chaque occurrence indépendamment une chaîne grasse et est choisi parmi un groupe alkyle ou alcényle ayant au moins 1 atome de carbone et notamment de 4 à 24 atomes de carbone ;
- $R'^2$, représente à chaque occurrence indépendamment un radical hydrocarboné comprenant de 1 à 52 atomes de carbone ;
- $R'^3$, représente à chaque occurrence indépendamment un groupement organique comprenant au moins un atome choisi les atomes de carbone, d'hydrogène, d'azote, à la condition que $R'^3$ comprend au moins 3 atomes de carbone ;
- $R'^4$ représente à chaque occurrence indépendamment : un atome d'hydrogène, un groupes alkyle en comprenant de 1 à 10 atomes de carbone,

ou une liaison directe à au moins un groupe choisi parmi $R'^3$ et un autre $R'^4$ de sorte que quand ledit groupe est un autre $R'^4$, l'atome d'azote auquel sont liés à la fois $R'^3$ et $R'^4$ fasse partie d'une structure hétérocyclique définie par $R'^4$-N-$R'^3$, à la condition qu'au moins 50% des $R'^4$ représentent un atome d'hydrogène, et

- L représente un groupe de liaison tel que défini plus haut dans la description, éventuellement substitué par au moins

un groupe R'1 tel que défini ci-dessus.

Selon un mode de réalisation, ces polymères sont choisis parmi les polymères de formule (A) dans laquelle le groupe de liaison L représente un groupe ester

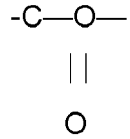

Ces polymères sont plus spécialement ceux décrits dans le document US-A-5783657 de la société Union Camp. Chacun de ces polymères satisfait notamment à la formule (B) suivante :

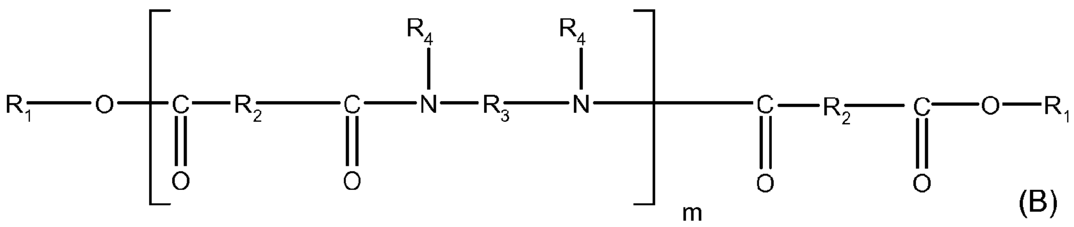

(B)

dans laquelle :

- m désigne un nombre entier de motifs amide tel que le nombre de groupes ester représente de 10 % à 50 % du nombre total des groupes ester et amide ;
- $R^1$ est à chaque occurrence indépendamment un groupe alkyle ou alcényle ayant au moins 4 atomes de carbone et notamment de 4 à 24 atomes de carbone ;
- $R^2$ représente à chaque occurrence indépendamment un groupe hydrocarboné en $C_4$ à $C_{42}$ à condition que 50 % des groupes $R^2$ représentent un groupe hydrocarboné en $C_{30}$ à $C_{42}$ ;
- $R^3$ représente à chaque occurrence indépendamment un groupe organique pourvu d'au moins 2 atomes de carbone, d'atomes d'hydrogène et optionnellement d'un ou plusieurs atomes d'oxygène ou d'azote ;
- et $R^4$ représente à chaque occurrence indépendamment un atome d'hydrogène, un groupe alkyle en $C_1$ à $C_{10}$ ou une liaison directe à $R^3$ ou à un autre $R^4$ de sorte que l'atome d'azote auquel sont liés à la fois $R^3$ et $R^4$ fasse partie d'une structure hétérocyclique définie par $R^4$-N-$R^3$, avec au moins 50 % des $R^4$ représentant un atome d'hydrogène.

Dans le cas particulier de la formule (B), les chaînes grasses terminales éventuellement fonctionnalisées au sens de l'invention sont des chaînes terminales liées au dernier atome d'azote, du squelette polyamide.

En particulier, les groupes ester de la formule (B), qui font partie des chaînes grasses terminales et/ou pendantes au sens de l'invention, représentent de 15 à 40 % du nombre total des groupes ester et amide et mieux de 20 à 35 %. De plus, m représente avantageusement un nombre entier allant de 1 à 5 et mieux supérieur à 2.
De préférence, $R^1$ est un groupe alkyle en $C_{12}$ à $C_{22}$ et de préférence en $C_{16}$ à $C_{22}$. Avantageusement, $R^2$ peut être un groupe hydrocarboné (alkylène) en $C_{10}$ à $C_{42}$. De préférence, 50 % au moins et mieux au moins 75 % des $R^2$ sont des groupes ayant de 30 à 42 atomes de carbone. Les autres $R^2$ sont des groupes hydrogénés en $C_4$ à $C_{19}$ et même en $C_4$ à $C_{12}$.
De préférence, $R^3$ représente un groupe hydrocarboné en $C_2$ à $C_{36}$ ou un groupe polyoxyalkyléné et $R^4$ représente un atome d'hydrogène. De préférence, $R^3$ représente un groupe hydrocarboné en $C_2$ à $C_{12}$.

Les groupes hydrocarbonés peuvent être des groupes linéaires, cycliques ou ramifiés, saturés ou insaturés. Par ailleurs, les groupes alkyle et alkylène peuvent être des groupes linéaires ou ramifiés, saturés ou non.

En général, les polymères de formule (B) se présentent sous forme de mélanges de polymères, ces mélanges pouvant en outre contenir un produit de synthèse correspondant à un composé de formule (B) où n vaut 0, c'est-à-dire un diester.

A titre d'exemple de polyamides de formule (B) utilisables dans la composition selon l'invention, on peut citer les produits commerciaux vendus par la société Arizona Chemical sous les noms Uniclear 80 et Uniclear 100 VG qui sont vendus respectivement sous forme de gel à 80 % (en matière active) dans une huile minérale et à 100 % (en

matière active). Ils ont un point de ramollissement de 88°C à 94°C. Ces produits commerciaux sont un mélange de copolymères d'un diacide en $C_{36}$ condensé sur l'éthylène diamine, de masse moléculaire moyenne en poids d'environ 6000. Les groupes ester terminaux résultent de l'estérification des terminaisons d'acide restantes par l'alcool cétylique, stéarylique ou leurs mélanges (appelés aussi alcool cétylstéarylique).

A titre de polyamides répondant à la formule générale (A), on peut également citer les polymères comprenant au moins une chaîne grasse terminale liée au squelette polymérique par au moins un groupe de liaison amide tertiaire (appelés aussi amide terminated polyamide ou ATPA).
De tels polymères sont disponibles par exemple sous la référence Sylvaclear A 200 V de la société Arizona Chemical. De tels polymères sont décrits par exemple dans le document US 6503522.
Selon un mode de réalisation, le polyamide de formule (A) peut également comprendre au moins une chaîne grasse terminale liée au squelette polymérique par au moins un groupe de liaison éther ou polyéther (il est dit alors ether terminated poly(ether)amide). De tels polymères sont décrits par exemple dans le document US 6 399 713.

Comme polyamide utilisable dans l'invention, on peut encore citer les résines polyamides résultant de la condensation d'un acide di-carboxylique aliphatique et d'une diamine (incluant les composés ayant plus de 2 groupes carbonyle et 2 groupes amine), les groupes carbonyle et amine de motifs unitaires adjacents étant condensés par une liaison amide. Ces résines polyamides sont notamment celles commercialisées sous la marque Versamid® par les sociétés General Mills, Inc. et Henkel Corp. (Versamid 930, 744 ou 1655) ou par la société Olin Mathieson Chemical Corp., sous la marque Onamid® notamment Onamid S ou C. Ces résines ont une masse moléculaire moyenne en poids allant de 6000 à 9000. Pour plus d'information sur ces polyamides, on peut se référer aux documents US 3645705 et US 3148125. Plus spécialement, on utilise les Versamid® 930 ou 744.

Il est aussi possible d'utiliser un poly(ester-amide) à extremités ester (ester-terminated poly(ester-amide) ou ETPEA), comme par exemple ceux dont la préparation est décrite dans le document US 6 552 160. Ce polyamide est par exemple le Sylvaclear C 75 V d'Arizona Chemical.

On peut aussi utiliser les polyamides vendus par la société Arizona Chemical sous les références Uni-Rez (2658, 2931, 2970, 2621, 2613, 2624, 2665, 1554, 2623, 2662) et le produit vendu sous la référence Macromelt 6212 par la société Henkel. De tels polymères sont décrits par exemple dans le document US 5500209.

Il est aussi possible d'utiliser des résines de polyamides issues de légumes comme celles décrites dans les brevets US 5783657 et US 5998570.

Le polyamide a avantageusement une température de ramollissement supérieure à 65°C et pouvant aller jusqu'à 190°C. De préférence, il présente une température de ramollissement allant de 70 à 130°C et mieux de 80 à 105°C. Le polyamide est en particulier un polymère non cireux.

De préférence, le polyamide répond à la formule (B) mentionnée précédemment.

## C/ Copolymère d'oléfine

La composition selon l'invention comprend au moins un copolymère d'oléfine choisi de préférence parmi les copolymères d'oléfine amorphes.

Par copolymère d'oléfine au sens de la présente demande, on entend tout copolymère formé par polymérisation d'au moins une oléfine et d'un autre monomère additionnel différent de ladite oléfine.

L'oléfine peut être notamment un monomère à insaturation éthylénique.
Comme exemple d'oléfine, on peut citer les monomères de carbure éthylénique, ayant notamment une ou deux insaturation éthylénique, ayant de 2 à 5 atomes de carbone tels que l'éthylène, le propylène, le butadiène, l'isoprène.

Par polymère amorphe, on entend un polymère qui n'a pas de forme cristalline. Ce polymère peut être également filmogène, c'est-à-dire qu'il est capable de former un film lors de son application sur la peau.

Le copolymère d'oléfine peut être notamment un copolymère dibloc, tribloc, multibloc, radial, étoile, ou leurs mélanges.
De tels composés sont décrits par exemple dans la demande US-A-2002/005562 et dans le brevet US-A-5 221 534
On choisit avantageusement un copolymère bloc amorphe de styrène et d'oléfine.
Le copolymère bloc est de préférence hydrogéné pour réduire les insaturations éthyléniques résiduelles après la polymérisation des monomères.
En particulier, le copolymère bloc hydrocarboné est un copolymère, éventuellement hydrogéné, à blocs styrène et à blocs éthylène/alkylène en $C_3$-$C_4$.
Comme copolymère dibloc, de préférence hydrogéné, on peut citer les copolymères de styrène-éthylène/propylène, les copolymères de styrène-éthylène/butadiène, les copolymères de styrène-éthylène/butylène. Des polymères diblocs sont notamment vendus sous la dénomination Kraton® G1701 E par la société Kraton Polymers.
Comme copolymère tribloc, de préférence hydrogéné, on peut citer les copolymères de styrène-éthylène/propylène-styrène, les copolymères de styrène-éthylène/butadiène-styrène, les copolymères de styrène-isoprène-styrène, les copolymères de styrène-butadiène-styrène.

Des polymères triblocs sont notamment vendus sous les dénominations Kraton® G1650E, Kraton® G1652, Kraton® D1101, Kraton® D1102, Kraton® D1160 par la société Kraton Polymers.
On peut notamment utiliser un copolymère tribloc styrène-éthylène/butylène- styrène.
Selon un mode de réalisation de l'invention, on peut notamment utiliser une mélange d'un copolymère tribloc styrène-butylène/éthylène-styrène et d'un copolymère dibloc styrène-éthylène/butylène, notamment vendus sous la dénomination Kraton® G1657M par la société Kraton Polymers.
On peut également utiliser un mélange de copolymère hydrogéné tribloc styrène-butylène/éthylène-styrène et de polymère étoile hydrogéné éthylène-propylène-styrène, un tel mélange étant notamment dans l'isododécane. De tels mélanges sont par exemple vendus par la société PENRECO sous les dénominations commerciales VERSAGEL® M5960 et VERSAGEL® M5670.

**D/ Organopolysiloxanes élastomériques**

On entend par « élastomère » un matériau souple, déformable ayant des propriétés viscoélastiques et notamment la consistance d'une éponge ou d'une sphère souple. Son module d'élasticité est tel que ce matériau résiste à la déformation et possède une capacité limitée à l'extension et à la contraction. Ce matériau est capable de retrouver sa forme originelle suite à un étirement. Cet élastomère est formé de chaînes polymériques de haut poids moléculaire dont la mobilité est limitée par un réseau uniforme de points de réticulation.
Les organopolysiloxanes élastomères utilisés dans la composition selon l'invention sont de préférence solides et peuvent être partiellement ou totalement réticulés. Inclus dans une phase huileuse, ils se transforment, selon le taux de phase huileuse utilisé, d'un produit d'aspect spongieux lorsqu'ils sont utilisés en présence de faibles teneurs en phase huileuse en un gel homogène en présence de quantités de phase huileuse plus élevées. La gélification de la phase huileuse par ces élastomères peut être totale ou partielle.
Les élastomères de l'invention peuvent être véhiculés sous forme de gel constitué d'un organopolysiloxane élastomère, incluant au moins une huile hydrocarbonée et/ou une huile de silicone et/ou une huile fluorée. Aussi, la phase huileuse associée à l'organopolysiloxane élastomère peut être constituée de cette ou ces huiles.
Les organopolysiloxanes élastomères utilisés selon l'invention peuvent être choisis parmi les polymères réticulés décrits dans la demande EP-A-0295886. Selon cette demande, ils sont obtenus par réaction d'addition et de réticulation, en présence d'un catalyseur du type platine, d'au moins :

- (a) un organopolysiloxane ayant au moins deux groupes alcényle inférieurs par molécule, ces groupes alcényle comportant deux à six atomes de carbone ; et
- (b) un organopolysiloxane ayant au moins deux atomes d'hydrogène liés à un atome de silicium par molécule.

Les organopolysiloxanes élastomères utilisés dans la composition de l'invention peuvent aussi être choisis parmi ceux décrits dans le brevet US-A-5,266,321. Selon ce brevet, ils sont choisis notamment parmi :

- i) les organopolysiloxanes comprenant des motifs $R_2SiO$ et $RSiO_{1,5}$ et éventuellement des motifs $R_3SiO_{0,5}$ et/ou $SiO_2$, dans lesquels les radicaux R, indépendamment les uns des autres, représentent un hydrogène, un radical alkyle tel que méthyle, éthyle ou propyle, un radical aryle tel que phényle ou tolyle, un groupe aliphatique insaturé tel que vinyle, le rapport en poids des motifs $R_2SiO$ sur les motifs $RSiO_{1,5}$ allant de 1/1 à 30/1 ;
- ii) les organopolysiloxanes insolubles et gonflables dans l'huile de silicone, obtenus par addition d'un organohydrogénopolysiloxane (1) et d'un organopolysiloxane (2) ayant des groupes aliphatiques insaturés de telle sorte que la quantité d'hydrogène ou de groupes aliphatiques insaturés dans respectivement (1) et (2) soit comprise entre 1 et 20 moles % lorsque l'organopolysiloxane est non cyclique et entre 1 et 50 moles % lorsque l'organopolysiloxane est cyclique.

Les organopolysiloxanes élastomères utilisés dans la composition de l'invention peuvent être par exemple ceux commercialisés sous les noms KSG 6 par la société Shin-Etsu ; Trefil E-505C ou Trefil E-506C par la société Dow-Corning ; Gransil (SR-CYC, SR DMF10, SR-DC556) par la société Grant Industries, ou ceux commercialisés sous forme de gels déjà constitués : KSG 15, KSG 16, KSG 17, KSG 18, KSG 26A, KSG 26B, KSG 41, KSG 42, KSG 43, KSG 44 de la société Shin-Etsu ; Gransil SR 5CYC gel, Gransil SR DMF 10 gel, Gransil SR DC556 gel de la société Grant Industries ; 1229-02-167 et 1229-02-168 de la société General Electric. On peut aussi utiliser un mélange d'élastomères de silicone, et notamment un mélange de ces produits commerciaux.

**E/ Polymères semi-cristallins**

On entend par polymère des composés comportant au moins deux motifs, de préférence au moins 3 motifs et

plus spécialement au moins 10 motifs de répétition. Par "polymère semi-cristallin", on entend des polymères comportant une partie cristallisable, une chaîne pendante cristallisable ou une séquence cristallisable dans le squelette, et une partie amorphe dans le squelette et présentant une température de changement de phase réversible du premier ordre, en particulier de fusion (transition solide-liquide). Lorsque la partie cristallisable est sous forme d'une séquence cristallisable du squelette polymérique, la partie amorphe du polymère est sous forme de séquence amorphe ; le polymère semi-cristallin est dans ce cas un copolymère séquencé par exemple du type dibloc, tribloc ou multibloc, comportant au moins une séquence cristallisable et au moins une séquence amorphe. Par "séquence", on entend généralement au moins 5 motifs de répétition identiques. La ou les séquences cristallisables sont alors de nature chimique différente de la ou des séquences amorphes.
Le polymère semi-cristallin a une température de fusion supérieure ou égale à 30°C (notamment allant de 30°C à 80°C), de préférence allant de 30°C à 60°C. Cette température de fusion est une température de changement d'état du premier ordre.
Cette température de fusion peut être mesurée par toute méthode connue et en particulier à l'aide d'un calorimètre à balayage différentiel (D.S.C).
De façon avantageuse, le ou les polymères semi-cristallins auxquels s'applique l'invention présentent une masse moléculaire moyenne en nombre supérieure ou égale à 1 000. De façon avantageuse, le ou les polymères semi-cristallins de la composition de l'invention ont une masse moléculaire moyenne en nombre $\overline{M}n$ allant de 2 000 à 800 000, de préférence de 3 000 à 500 000, mieux de 4 000 à 150 000, notamment inférieure à 100 000, et mieux de 4 000 à 99 000. De préférence, ils présentent une masse moléculaire moyenne en nombre supérieure à 5 600, allant par exemple de 5 700 à 99 000.Par "chaîne ou séquence cristallisable", on entend au sens de l'invention une chaîne ou séquence qui si elle était seule passerait de l'état amorphe à l'état cristallin, de façon réversible, selon qu'on est au-dessus
ou en dessous de la température de fusion. Une chaîne au sens de l'invention est un groupement d'atomes, pendant ou latéral par rapport au squelette du polymère. Une séquence est un groupement d'atomes appartenant au squelette, groupement constituant un des motifs répétitif du polymère. Avantageusement, la "chaîne pendante cristallisable" peut être chaîne comportant au moins 6 atomes de carbone.
Le polymère semi-cristallin peut être choisi parmi les copolymères séquencés comportant au moins une séquence cristallisable et au moins une séquence amorphe, les homopolymères et les copolymères portant au moins une chaîne latérale cristallisable par motif de répétition, leurs mélanges.
De tels polymères sont décrits par exemple dans le document EP 1396259.
Selon un mode plus particulier de réalisation de l'invention, le polymère est issu d'un monomère à chaîne cristallisable choisi parmi les (méth)acrylates d'alkyle saturés en $C_{14}$ à $C_{22}$.
A titre d'exemple particulier de polymère semi-cristallin structurant utilisable dans la composition selon l'invention, on peut citer les produits Intelimer® de la société Landec décrits dans la brochure "Intelimer® polymers", Landec IP22 (Rev. 4-97). Ces polymères sont sous forme solide à température ambiante (25°C).

**<u>F/ Ester de dextrine et d'acide(s) aras</u>**

La composition selon l'invention comprend au moins un ester de dextrine et d'acide(s) gras. Plus particulièrement, il s'agit d'un mono-ou poly-ester de dextrine et d'au moins un acide gras et notamment répondant à la formule (C) :

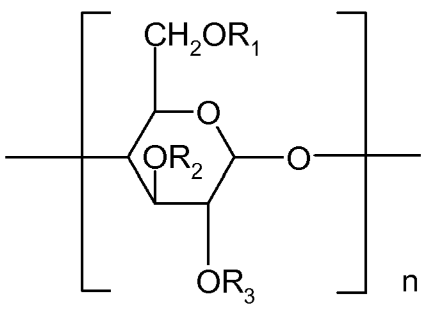

(C)

dans laquelle :

- n est un entier allant de 3 à 200, notamment allant de 20 à 150, et en particulier allant de 25 à 50,
- les radicaux $R_1$, $R_2$ et $R_3$, identiques ou différents, sont choisis parmi l'hydrogène ou un groupement acyle (R-CO-) dans lequel le radical R est un groupement hydrocarboné, linéaire ou ramifié, saturé ou insaturé, possédant de 5

à 29, en particulier de 7 à 21, notamment de 11 à 19, plus particulièrement de 13 à 17, voire 15, atomes de carbone, sous réserve qu'au moins un desdits radicaux $R_1$, $R_2$ ou $R_3$ est différent de l'hydrogène.

En particulier, $R_1$, $R_2$ et $R_3$ peuvent représenter l'hydrogène ou un groupement acyle (R-CO-) dans lequel R est un radical hydrocarboné tel que défini précédemment, sous réserve qu'au moins deux desdits radicaux $R_1$, $R_2$ ou $R_3$ sont identiques et différents de l'hydrogène.

L'ensemble des radicaux $R_1$, $R_2$ et $R_3$ peuvent figurer un groupement acyle (R-CO) identique

ou différent et notamment identique.

En particulier, n est avantageusement varie de 25 à 50, notamment est égal à 38 dans la formule générale (C) de l'ester selon l'invention.

Notamment lorsque les radicaux $R_1$, $R_2$ et/ou $R_3$, identiques ou différents figurent un groupement acyle (R-CO), ceux-ci peuvent être choisis parmi les radicaux caprylique, caprique, laurique, myristique, palmitique, stéarique, arachique, béhénique, isobutyrique, isovalérique, éthyl-2 butyrique, éthylméthylacétique, isoheptanoïque, éthyl-2 hexanoïque, isononanoïque, isodécanoïque, isotridécanoïque, isomyristique, isopalmitique, isostéarique, isoaracique, isohexanoïque, décénoïque, dodécenoïque, tetradécénoïque, myristoléïque, hexadécénoïque, palmitoléïque, oléïque, élaidique, asclépinique, gondoléïque, eicosènoïque, sorbique, linoléïque, linolénique, punicique, stéaridonique, arachidonique, stéarolique, eicosanyle, docosanoyle, et leurs mélanges.

De préférence, on utilise à titre d'ester de dextrine et d'acide(s) gras au moins un palmitate de dextrine. Celui-ci peut être utilisé seul ou en mélange avec d'autres esters.

Avantageusement, l'ester de dextrine et d'acide gras a un degré de substitution inférieur ou égal à 2,5 sur la base d'une unité glucose, notamment variant de 1,5 à 2,5, de préférence de 2 à 2,5. Le poids moléculaire moyen en poids de l'ester de dextrine peut être en particulier de 10 000 à 150 000, notamment de 12 000 à 100 000 et voire de 15 000 à 80 000

Des esters de dextrine, en particulier des palmitates de dextrine, sont disponibles commercialement sous la dénomination RHEOPEARL TL ou RHEOPEARL KL de la société Chiba Flour.

**<u>III/ Composés additionnels</u>**

**<u>Cires</u>**

La composition selon l'invention est exempte de cires.

Par « exempte de cire », on entend une composition comprenant moins de 5% en poids, de préférence moins de 3%, mieux moins de 2%, et encore mieux moins de 1% en poids de cire par rapport au poids total de la composition. De préférence encore, la composition ne comprend pas de cire.

La cire considérée dans le cadre de la présente invention est d'une manière générale un composé lipophile, solide à température ambiante (25 °C), à changement d'état solide/liquide réversible, ayant un point de fusion supérieur ou égal à 30 °C pouvant aller jusqu'à 200 °C et notamment jusqu'à 120 °C.

En portant la cire à l'état liquide (fusion), il est possible de la rendre miscible aux huiles et de former un mélange homogène microscopiquement, mais en ramenant la température du mélange à la température ambiante, on obtient une recristallisation de la cire dans les huiles du mélange.

En particulier, les cires peuvent présenter un point de fusion supérieur ou égal à 45 °C, et en particulier supérieur ou égal à 55°C.

Au sens de l'invention, la température de fusion correspond à la température du pic le plus endothermique observé en analyse thermique (DSC) telle que décrite dans la norme ISO 11357-3 ; 1999. Le point de fusion de la cire peut être mesuré à l'aide d'un calorimètre à balayage différentiel (DSC), par exemple le calorimètre vendu sous la dénomination « MDSC 2920 » par la société TA Instruments.

Le protocole de mesure est le suivant :

Un échantillon de 5 mg de cire disposé dans un creuset est soumis à une première montée en température allant de -20 °C à 100 °C, à la vitesse de chauffe de 10 °C/minute, puis est refroidi de 100 °C à -20 °C à une vitesse de refroidissement de 10 °C/minute et enfin soumis à une deuxième montée en température allant de -20 °C à 100 °C à une vitesse de chauffe de 5 °C/minute. Pendant la deuxième montée en température, on mesure la variation de la différence de puissance absorbée par le creuset vide et par le creuset contenant l'échantillon de cire en fonction de la température. Le point de fusion du composé est la valeur de la température correspondant au sommet du pic de la courbe représentant la variation de la différence de puissance absorbée en fonction de la température.

Les cires peuvent être d'origine animale, végétale, minérale ou de synthèse et leurs mélanges.

Les cires peuvent présenter généralement une dureté allant de 0,01 MPa à 15 MPa, notamment supérieure à 0,05 MPa et en particulier supérieure à 0,1 MPa.

La dureté est déterminée par la mesure de la force en compression mesurée à 20 °C à l'aide du texturomètre vendu

sous la dénomination TA-XT2 par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm.

Le protocole de mesure est le suivant :

La cire est fondue à une température égale au point de fusion de la cire + 10 °C. La cire fondue est coulée dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure de la dureté ou du collant.
Le mobile du texturomètre est déplacé à la vitesse de 0,1 mm/s, puis pénètre dans la cire jusqu'à une profondeur de pénétration de 0,3 mm. Lorsque le mobile a pénétré dans la cire à la profondeur de 0,3 mm, le mobile est maintenu fixe pendant 1 seconde (correspondant au temps de relaxation) puis est retiré à la vitesse de 0,5 mm/s.
La valeur de la dureté est la force de compression maximale mesurée divisée par la surface du cylindre du texturomètre en contact avec la cire.
Les cires peuvent présenter généralement une masse moléculaire moyenne en poids (ou poids moléculaire) inférieure ou égale à 3000, mieux inférieure ou égale à 2500, et encore mieux, inférieure ou égale à 2000.

A titre illustratif des cires, on peut notamment citer les cires hydrocarbonées comme la cire d'abeille, la cire de lanoline, et les cires d'insectes de Chine; la cire de son de riz, la cire de Carnauba, la cire de Candellila, la cire d'Ouricury, la cire d'Alfa, la cire de berry, la cire de shellac, la cire du Japon et la cire de sumac; la cire de montan, les cires d'orange et de citron, les cires microcristallines, les paraffines et l'ozokérite; les cires de polyéthylène, les cires obtenues par la synthèse de Fisher-Tropsch et les copolymères cireux ainsi que leurs esters.
On peut aussi citer des cires obtenues par hydrogénation catalytique d'huiles animales ou végétales ayant des chaînes grasses, linéaires ou ramifiées, en $C_8$-$C_{32}$. Parmi celles-ci, on peut notamment citer l'huile de jojoba isomérisée telle que l'huile de jojoba partiellement hydrogénée isomérisée trans fabriquée ou commercialisée par la société DESERT WHALE sous la référence commerciale Iso-Jojoba-50®, l'huile de tournesol hydrogénée, l'huile de ricin hydrogénée, l'huile de coprah hydrogénée, l'huile de lanoline hydrogénée, et le tétrastéarate de di-(triméthylol-1,1,1 propane) vendu sous la dénomination de Hest 2T-4S® par la société HETERENE.
On peut encore citer les cires de silicone, les cires fluorées.
On peut également utiliser les cires obtenues par hydrogénation d'huile de ricin estérifiée avec l'alcool cétylique vendues sous les dénominations de Phytowax ricin 16L64® et 22L73® par la société SOPHIM. De telles cires sont décrites dans la demande FR-A- 2792190.
On peut citer également les cires dite cire collantes c'est-à-dire possédant un collant supérieur ou égal à 0,1 N.s et une dureté inférieure ou égale à 3,5 MPa.
La cire collante utilisée peut posséder notamment un collant allant de 0,1 N.s à 10 N.s, en, particulier allant de 0,1 N.s à 5 N.s, de préférence allant de 0,2 à 5 N.s et mieux allant de 0,3 à 2 N.s.
Le collant de la cire est déterminé par la mesure de l'évolution de la force (force de compression) en fonction du temps, à 20 °C selon le protocole indiqué précédemment pour la dureté.
Pendant le temps de relaxation de 1 s, la force (force de compression) décroît fortement jusqu'à devenir nulle puis, lors du retrait du mobile, la force (force d'étirement) devient négative pour ensuite croître à nouveau vers la valeur 0. Le collant correspond à l'intégrale de la courbe de la force en fonction du temps pour la partie de la courbe correspondant aux valeurs négatives de la force. La valeur du collant est exprimée en N.s.
La cire collante pouvant être utilisée a généralement une dureté inférieure ou égale à 3,5 MPa, en particulier allant de 0,01 MPa à 3,5 MPa, notamment allant de 0,05 MPa à 3 MPa.
Comme cire collante, on peut utiliser un (hydroxystéaryloxy)stéarate d'alkyle en $C_{20}$-$C_{40}$ (le groupe alkyle comprenant de 20 à 40 atomes de carbone), seul ou en mélange.
Une telle cire est notamment vendue sous les dénominations « Kester Wax K 82 P® », « Hydroxypolyester K 82 P® » et « Kester Wax K 80 P® » par la société KOSTER KEUNEN.

## Polymère filmogène

La composition selon l'invention peut comprendre au moins un polymère filmogène.
Le polymère filmogène peut être présent dans la composition selon l'invention en une teneur en matières sèches (ou matières actives) allant de 0,1 % à 30 % en poids par rapport au poids total de la composition, de préférence de 0,5 % à 20 % en poids, et mieux de 1 % à 15 % en poids.
Dans la présente invention, on entend par « polymère filmogène », un polymère apte à former à lui seul ou en présence d'un agent auxiliaire de filmification, un film continu et adhérent sur un support, notamment sur les matières kératiniques.

La composition selon l'invention peut comprendre au moins un polymère filmogène liposoluble (c'est-à-dire soluble dans une phase grasse liquide comprenant des huiles ou solvants organiques tels que ceux décrits précédemment) ou lipophile (c'est-à-dire compatible avec une phase grasse liquide comprenant des huiles ou solvants organiques tels que ceux décrits précédemment).

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester) et d'au moins un autre monomère qui peut être un ester vinylique (différent de l'ester vinylique déjà présent), une α-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les copolymères liposolubles, et en particulier ceux résultant de copolymérisation d'esters vinyliques ayant de 9 à 22 atomes de carbone ou d'acrylates ou de méthacrylates d'alkyle, les radicaux alkyles ayant de 10 à 20 atomes de carbone.

De tels copolymères liposolubles peuvent être choisis parmi les copolymères de polystéarate de vinyle, de polystéarate de vinyle réticulé à l'aide de divinylbenzène, de diallyléther ou de phtalate de diallyle, les copolymères de poly(méth) acrylate de stéaryle, de polylaurate de vinyle, de poly(méth)acrylate de lauryle, ces poly(méth)acrylates pouvant être réticulés à l'aide de diméthacrylate de l'éthylène glycol ou de tétraéthylène glycol.

Les copolymères liposolubles définis précédemment sont connus et notamment décrits dans la demande FR-A-2232303 ; ils peuvent avoir un poids moléculaire moyen en poids allant de 2.000 à 500.000 et de préférence de 4.000 à 200.000.

Comme polymères filmogènes liposolubles utilisables dans l'invention, on peut également citer les polyalkylènes et notamment les copolymères d'alcènes en C2-C20, comme le polybutène, les alkylcelluloses avec un radical alkyle linéaire ou ramifié, saturé ou non en C1 à C8 comme l'éthylcellulose et la propylcellulose, les copolymères de la vinylpyrolidone (VP) et notamment les copolymères de la vinylpyrrolidone et d'alcène en C2 à C40 et mieux en C3 à C20. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

On peut également citer les résines de silicone, généralement solubles ou gonflables dans les huiles de silicone, qui sont des polymères de polyorganosiloxanes réticulés. La nomenclature des résines de silicone est connue sous le nom de "MDTQ", la résine étant décrite en fonction des différentes unités monomèriques siloxane qu'elle comprend, chacune des lettres "MDTQ" caractérisant un type d'unité.

A titre d'exemples de résines polymethylsilsesquioxanes commercialement disponibles, on peut citer celles qui sont commercialisés :

par la société Wacker sous la référence Resin MK tels que la Belsil PMS MK :

par la société SHIN-ETSU sous les références KR-220L.

Comme résines siloxysilicates, on peut citer les résines trimethylsiloxysilicate (TMS) telles que celle commercialisées sous la référence SR1000 par la société General Electric ou sous la référence TMS 803 par la société Wacker. On peut encore citer les résines timéthylsiloxysilicate commercialisées dans un solvant tel que la cyclomethicone, vendues sous la dénomination "KF-7312J" par la société Shin-Etsu, "DC 749", "DC 593" par la société Dow Corning.

On peut aussi citer des copolymères de résines de silicone telles que celles citées ci-dessus avec des polydiméthylsiloxanes, comme les copolymères adhésifs sensibles à la pression commercialisés par la société Dow Corning sous la référence BIO-PSA et décrits dans le document US 5 162 410 ou encore les copolymères siliconés issus de la réaction

d'un résine de silicone, telle que celles décrite plus haut, et d'un diorganosiloxane tels que décrits dans le document WO 2004/073626.

Selon un mode de réalisation de l'invention, le polymère filmogène est un polymère éthylénique séquencé linéaire filmogène, qui comprend de préférence au moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.

Avantageusement, les première et deuxième séquences et du polymère séquencé sont incompatibles l'une avec l'autre.

De tels polymères sont décrits par exemple dans les documents EP 1411069 ou WO04/028488.

Le polymère filmogène lipophile ou liposoluble peut être également présent dans la composition sous la forme de particules en dispersion dans une phase solvant non aqueuse qui peut être celle de la composition selon l'invention. Les techniques de préparation de ces dispersions sont bien connues de l'homme du métier.

Comme exemples de dispersions non aqueuses de polymère filmogène, on peut citer les dispersion décrite par exemple dans le document EP 749 746 et notamment les particules de polymères acryliques, stabilisées en surface par un stabilisant, en dispersion dans une phase grasse (par exemple l'isododécane) comme le Mexomère PAP® de la société CHIMEX, les dispersions de particules d'un polymère éthylénique greffé, de préférence acrylique, dans une phase grasse liquide, le polymère éthylénique étant avantageusement dispersé en l'absence de stabilisant additionnel en surface des particules telles que décrite notamment dans le document WO 04/055081.

Le polymère filmogène peut également se présenter sous forme de particules en dispersion dans une phase aqueuse comme par exemple les dispersions acryliques vendues sous les dénominations Neocryl XK-90®, Neocryl A-1070®, Neocryl A-1090®, Neocryl BT-62®, Neocryl A-1079® et Neocryl A-523® par la société AVECIA-NEORESINS, Dow Latex 432® par la société DOW CHEMICAL, Daitosol 5000 AD® ou Daitosol 5000 SJ® par la société DAITO KASEY KOGYO; Syntran 5760® par la société Interpolymer ou encore les dispersions aqueuses de polyuréthane vendues sous les dénominations Neorez R-981® et Neorez R-974® par la société AVECIA-NEORESINS, les Avalure UR-405®, Avalure UR-410®, Avalure UR-425®, Avalure UR-450®, Sancure 875®, Sancure 861®, Sancure 878® et Sancure 2060® par la société GOODRICH, Impranil 85® par la société BAYER, Aquamere H-1511® par la société HYDROMER ; les sulfopolyesters vendus sous le nom de marque Eastman AQ® par la société Eastman Chemical Products, les dispersions vinyliques comme le Mexomère PAM® de la société CHIMEX et leurs mélanges.

La composition selon l'invention peut comprendre un agent plastifiant favorisant la formation d'un film avec le polymère filmogène. Un tel agent plastifiant peut être choisi parmi tous les composés connus de l'homme du métier comme étant susceptibles de remplir la fonction recherchée.

Matière colorante

La composition selon l'invention peut également comprendre au moins une matière colorante comme les matières pulvérulentes, les colorants liposolubles, les colorants hydrosolubles.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C Orange 5, le jaune quinoléine, le rocou.

Ces matières colorantes peuvent être présentes en une teneur allant de 0,01 à 30 % en poids par rapport au poids total de la composition.

Charges

La composition selon l'invention peut en outre comprendre au moins une charge.

Les charges peuvent être choisies parmi celles bien connues de l'homme du métier et couramment utilisées dans les compositions cosmétiques. Les charges peuvent être minérales ou organiques, lamellaires ou sphériques. On peut citer le talc, le mica, la silice, le kaolin, les poudres de polyamide comme le Nylon® commercialisé sous la dénomination Orgasol® par la société Atochem, de poly-β-alanine et de polyéthylène, les poudres de polymères de tétrafluoroéthylène

comme le Téflon®, la lauroyl-lysine, l'amidon, le nitrure de bore, les micro sphères creuses polymériques expansées telles que celles de chlorure de polyvinylidène/acrylonitrile comme celles commercialisées sous la dénomination d'Expancel® par la société Nobel Industrie, les poudres acryliques telles que celles commercialisées sous la dénomination Polytrap® par la société Dow Corning, les particules de polyméthacrylate de méthyle et les microbilles de résine de silicone (Tospearls® de Toshiba, par exemple), le carbonate de calcium précipité, le carbonate et l'hydro-carbonate de magnésium, l'hydroxyapatite, les microsphères de silice creuses (Silica Beads® de MAPRECOS), les microcapsules de verre ou de céramique, les savons métalliques dérivés d'acides organiques carboxyliques ayant de 8 à 22 atomes de carbone, et en particulier de 12 à 18 atomes de carbone, par exemple le stéarate de zinc, de magnésium ou de lithium, le laurate de zinc, le myristate de magnésium.
On peut également utiliser un composé susceptibles de gonfler à la chaleur et notamment des particules thermoexpansibles telles que les microsphères non expansées de copolymère de chlorure de vinylidène/d'acrylonitrile/méthacrylate de méthyle ou de copolymère d'homopolymère d'acrylonitrile comme par exemple celles commercialisées respectivement sous les références Expancel® 820 DU 40 et Expancel®007WU par la Société AKZO NOBEL. Les charges peuvent représenter de 0,1 à 25 %, en particulier de 1 à 20 % en poids par rapport au poids total de la composition.

<u>**Phase aqueuse**</u>

La composition selon l'invention peut comprendre de l'eau et/ou de(s) solvant(s) hydrosoluble(s) qui peuvent être introduits en tant que tels dans la formulation selon l'invention ou y être incorporés par le biais, d'un ou plusieurs ingrédients constituant ladite composition. Ainsi de l'eau peut être notamment introduite dans la composition par le biais de l'introduction de latex ou de pseudolatex, c'est-à-dire de dispersion aqueuse de particules de polymère.
Les solvant miscible à l'eau (miscibilité dans l'eau supérieure à 50 % en poids à 25 °C) sont notamment les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en $C_3$-$C_4$, les aldéhydes en $C_2$-$C_4$ et leur mélanges.
De façon avantageuse, la composition présente une teneur en eau /ou solvant(s) hydrosoluble(s) inférieure ou égale à 15% en poids par rapport au poids total de la composition, de préférence inférieure ou égale à 10% en poids, mieux inférieure ou égale à 5% en poids, mieux encore inférieure ou égale à 3% en poids
Selon un mode de réalisation, la composition selon l'invention est exempte d'eau (composition anhydre).

<u>**Fibres**</u>

La composition selon l'invention peut en outre comprendre des fibres qui permettent une amélioration de l'effet allongeant.
Par « fibre », il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2500, en particulier de 5 à 500, et plus particulièrement de 5 à 150.
Les fibres utilisables dans la composition de l'invention peuvent être des fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.
En particulier, les fibres ont une longueur allant de 1 μm à 10 mm, en particulier de 0,1 mm à 5 mm et plus particulièrement de 0,3 mm à 3,5 mm. Leur section peut être comprise dans un cercle de diamètre allant de 2 nm à 500 μm, en particulier allant de 100 nm à 100 μm et plus particulièrement de 1 μm à 50 μm. Le poids ou titre des fibres est souvent donné en denier
ou décitex et représente le poids en gramme pour 9 km de fil. Les fibres selon l'invention peuvent en particulier avoir un titre choisi dans la gamme allant de 0,15 à 30 deniers et notamment de 0,18 à 18 deniers.
Les fibres utilisables dans la composition de l'invention peuvent être choisies parmi les fibres rigides ou non rigides, elles peuvent être d'origine synthétique ou naturelle, minérales ou organiques.
Par ailleurs, les fibres peuvent être traitées ou non en surface, enrobées ou non, colorées ou non colorées.
A titre de fibres utilisables dans la composition selon l'invention, on peut citer les fibres non rigides telles que les fibres de polyamide (Nylon®) ou les fibres rigides telles que les fibres de polyimide-amide comme celles vendues sous les dénomination KERMEL®, KERMEL TECH® par la société RHODIA ou de poly-(p-phénylène-téréphtalamide) (ou d'aramide) notamment vendues sous la dénomination Kevlar® par la société DUPONT DE NEMOURS. Les fibres peuvent êtres présentes dans la composition selon l'invention en une teneur allant de 0,01 % à 10 % en poids, par rapport au poids total de la composition, en particulier de 0,1 % à 5 % en poids, et plus particulièrement de 0,3 % à 3 % en poids.

**Actifs cosmétiques**

Comme actifs cosmétiques pouvant être utilisés dans les compositions selon l'invention, on peut citer notamment des antioxydants, les conservateurs, les parfums, les neutralisants, émollients, des hydratants, des vitamines et des filtres en particulier solaires.

Bien entendu, l'homme du métier veillera à choisir les éventuels additifs complémentaires et/ou leur quantité de telle manière que les propriétés avantageuses de la composition selon l'invention ne soient pas, ou substantiellement pas, altérées par l'adjonction envisagée.

La composition avant foisonnement peut se présenter sous forme de suspension, de dispersion, de solution, de gel.

**Procédé de préparation**

Les compositions mises en oeuvre dans l'invention peuvent être préparées par des procédés de mélange, d'agitation ou de dispersion de gaz comprimés tels que l'air, les composés à base de chlorofluorocarbone, l'azote, le dioxyde de carbone, l'oxygène, l'hélium, un procédé de mélange et d'agitation en présence d'un agent moussant tel qu'un tensioactif.

En particulier, la composition est préparée par mélange, généralement à chaud, des ingrédients sous agitation puis foisonnement sous l'action d'un gaz, la gaz pouvant être introduit lors de l'étape de refroidissement de la composition, ou après préparation de la composition, par exemple à l'aide d'un foisonneur de type Mondomix, d'un batteur de type Kenwood, d'un échangeur à surface raclée ou d'un mélangeur dynamique (de type IMT par exemple). Le gaz est de préférence l'air ou l'azote.

La composition selon l'invention peut être conditionnée dans un récipient délimitant au moins un compartiment qui comprend ladite composition, ledit récipient étant fermé par un élément de fermeture. Le récipient peut être équipé d'un organe pour la distribution dudit produit. En particulier, le récipient peut être équipé d'une pompe.
Le récipient peut être un pot.

Le récipient peut être au moins pour partie réalisé en matériau thermoplastique. A titre d'exemples de matériaux thermoplastiques, on peut citer le polypropylène ou le polyéthylène. Alternativement, le récipient est réalisé en matériau non thermoplastique, notamment en verre ou en métal (ou alliage).

La composition peut être appliqué au doigt ou à l'aide d'un applicateur.
Le récipient est de préférence associé à un applicateur comportant au moins un élément d'application configuré pour appliquer la composition sur les matière kératiniques.

Selon un autre mode de réalisation avantageux, l'applicateur comprend un embout d'application.

**EXEMPLES**

Les exemples qui suivent sont présentés à titre illustratif et non limitatif de l'invention. Sauf indication contraire, les quantités sont données en pourcentage en poids.

**Exemples 1 à 5 : Rouges à lèvres sous forme de mousse**

On prépare les compositions de rouge à lèvres suivantes selon l'invention :

| | Exemple1 | Exemple 2 | Exemple 3 | Exemple 4 | Exemple 5 |
|---|---|---|---|---|---|
| Copolymère polyamide/ polydiméthylsiloxane (n = 100) (DC 2-8179 de Dow Corning) | 25 | 19.74 | 24.2 | | |
| Copolymère polyamide/ polydiméthylsiloxane (n = 15) (DC 2-8178 de Dow Corning) | | | | 10 | |
| Palmitate de dextrine (RHEOPEARL KL de Chiba Flour) | | | | | 15 |
| Isononanoate d'isononyle | Qsp 100 | Qsp 100 | Qsp 100 | Qsp 100 | Qsp 100 |

(suite)

| | Exemple1 | Exemple 2 | Exemple 3 | Exemple 4 | Exemple 5 |
|---|---|---|---|---|---|
| Red 7 | 5 | 0.972 | 1.51 | 5 | 5 |
| Dioxyde de titane | | 0.432 | 1.82 | | |
| Oxyde de fer brun,jaune | | 2.052 | | | |
| Oxyde de fer noir | | | 1.36 | | |
| Yellow 5 lake | | 1.83 | | | |
| Red 27 lake | | | 1.26 | | |
| Yellow 6 lake | | | 2.05 | | |
| Blue 1 lake | | 0.432 | | | |
| Mica et Titanium dioxide | | 6.042 | | | |
| Mica et Titanium dioxide | | 1.08 | | | |
| Mica et Titanium dioxide | | 2.16 | | | |

*Mode opératoire*

Dans un bécher double enveloppe, on chauffe le structurant huileux, l'huile et les pigments à 80°C sous agitation de type pale défloculeuse.
Le mélange fondu est versé dans un foisonneur type « Mondomix » à l'aide d'une pompe péristaltique à un débit de 6kg/h ; la température d'entrée du mélange affiche 57°C et le produit est foisonné dans la tête mélangeuse qui est thermostatée à 20°C

A la sortie du foisonneur, la mousse, dont la température est de 30°C, est conditionnée dans des pots qui sont immédiatement placés pendant 20 minutes dans une enceinte thermostatée à -28°C.

Pour chacune des compositions, on a mesuré les paramètres de densité et de taux de foisonnement selon les protocoles décrits plus haut.

Les résultats sont présentés dans le tableau suivant :

| | Exemple1 | Exemple 2 | Exemple 3 | Exemple 4 | Exemple 5 |
|---|---|---|---|---|---|
| **Densité avant foisonnement** | 0.89 | 0.91 | 0.9 | 0.85 | 0.93 |
| **Densité après foisonnement** | 0.52 | 0.39 | 0.33 | 0.7 | 0.73 |
| **Taux de foisonnement** | 71% | 133% | 173% | 21% | 27% |

**<u>Exemple 6 : Fond de teint</u>**

| | |
|---|---|
| Copolymère polyamide/polydiméthylsiloxane<br>(n = 100)<br>(DC 2-8179 de Dow Corning) | 17.5 |
| Isononanoate d'isononyle | Qsp 100 |
| Talc | 9.9 |
| Poudre de nylon | 9.9 |
| Microsphère de copolymère chlorure de vinylidene/acrylonitrile/PMMA (Expancel 551 DE20 D60 d'EXPANCEL) | 0.2 |
| Dioxyde de titane | 7.91 |
| Oxyde de fer noir | 0.23 |

(suite)

| | |
|---|---|
| Oxyde de fer jaune | 1.4 |
| Oxyde de fer brun | 0.46 |

Le mode opératoire utilisé est le même que pour les compositions des exemples 1 à 5 ci-dessus.
On a mesuré les paramètres de densité et de taux de foisonnement de la composition selon les protocoles décrits plus haut.

| | |
|---|---|
| Densité avant foisonnement | 0.97 |
| Densité après foisonnement | 0.52 |
| Taux de foisonnement | 87% |

**Exemples 7 à 10 : Compositions non colorées**

On a préparé les compositions suivantes :

| | **Exemple 7** | **Exemple 8** | **Exemple 9** | **Exemple 10** |
|---|---|---|---|---|
| Copolymère polyamide/polydiméthylsiloxane (n = 100) (DC 2-8179 de Dow Corning) | 26.3 | 20 | 15 | 25 |
| Isononanoate d'isononyle | 73.7 | 80 | 85 | |
| Diméthicone (DC Fluid 200, 5cSt de Dow Corning) | | | | 75 |

Le mode opératoire utilisé est le même que pour les compositions des exemples 1 à 5 ci-dessus.
On a mesuré les paramètres de densité et de taux de foisonnement de chaque composition selon les protocoles décrits plus haut.

| | **Exemple 7** | **Exemple 8** | **Exemple 9** | **Exemple 10** |
|---|---|---|---|---|
| **Densité avant foisonnement** | **0.9** | **0.89** | **0.88** | **0.95** |
| **Densité après foisonnement** | **0.39** | **0.42** | **0.39** | **0.45** |
| **Taux de foisonnement** | **131%** | **112%** | **126%** | **111%** |

**Exemples 11 à 13 : Compositions non colorées**

On a préparé les compositions suivantes :

| | **Exemple 11** | **Exemple 12** | **Exemple 13** |
|---|---|---|---|
| Polyamide à groupes ester terminaux ("UNICLEAR® 100"V d'Arizona Chemical) | 20 | | |
| Copolymère tribloc styrène/ethylène-butylène/styrène (KRATON G 1650 E de KRATON POLYMERS) | | | 5 |
| Palmitate de dextrine (RHEOPEARL TL2 de Chiba Flour) | | 20 | |
| Isononanoate d'isononyle | 80 | 80 | |
| Huile de parléam | | | 95 |

Le mode opératoire utilisé est le même que pour les compositions des exemples 1 à 5 ci-dessus.
On a mesuré les paramètres de densité et de taux de foisonnement de chaque composition selon les protocoles

décrits plus haut.

| | Exemple 11 | Exemple 12 | Exemple 13 |
|---|---|---|---|
| **Densité avant foisonnement** | 0.85 | 0.91 | 0.8 |
| **Densité après foisonnement** | 0.75 | 0.73 | 0.68 |
| **Taux de foisonnement** | 15% | 25% | 18% |

**Exemples 14 et 15 :**

| | Exemple 14 | Exemple 15 |
|---|---|---|
| Copolymère polyamide/polydiméthylsiloxane<br>(n = 100)<br>(DC 2-8179 de Dow Corning) | 20 | 20 |
| Copolymère polyamide/polydiméthylsiloxane (n = 15)<br>(DC 2-8178 de Dow Corning) | 5 | |
| Palmitate de dextrine (RHEOPEARL TL2 de Chiba Flour) | | 5 |
| Isononanoate d'isononyle | 75 | 75 |

Le mode opératoire utilisé est le même que pour les compositions des exemples 1 à 5 ci-dessus.
On a mesuré les paramètres de densité et de taux de foisonnement de chaque composition selon les protocoles décrits plus haut.

| | Exemple 14 | Exemple 15 |
|---|---|---|
| **Densité avant foisonnement** | **0.91** | **0.92** |
| **Densité après foisonnement** | **0.40** | **0.37** |
| **Taux de foisonnement** | **128%** | **163%** |

## Revendications

1. Composition cosmétique sous forme de mousse comprenant une phase huileuse continue et au moins un agent structurant de la phase huileuse, ladite composition étant exempte de cire.

2. Composition selon la revendication 1, **caractérisée en ce qu'**elle présente une densité inférieure ou égale à 0,95.

3. Composition selon la revendication 1 ou 2, **caractérisée en ce qu'**elle présente une densité supérieure ou égale à 0,2, et mieux supérieure ou égale à 0,3 .

4. Composition selon l'une des revendications 1 à 3, **caractérisée en ce qu'**elle présente un taux de foisonnement allant de 10% à 300%, de préférence de 30% à 250% et mieux de 40% à 200%.

5. Composition selon l'une des revendications précédentes, **caractérisée en ce que** le ou les agents structurants de la phase huileuse sont présents en une teneur allant de 0,1 à 60 % en poids par rapport au poids total de la composition, mieux de 0,5 à 50 % en poids et encore mieux de 1 à 40% en poids.

6. Composition selon l'une des revendications précédentes, **caractérisée en ce que** l'agent structurant de la phase huileuse est choisi parmi :

   A/ les polymères siliconés comportant au moins un motif comprenant :

1) des polyorganosiloxanes comportant au moins deux groupes capables d'établir des interactions hydrogène, ces deux groupes étant situés dans la chaîne du polymère, et/ou
2) des polyorganosiloxanes comportant au moins deux groupes capables d'établir des interactions hydrogène, ces deux groupes étant situés sur des greffons ou ramifications,

lesdits groupes capables d'établir des interactions hydrogène étant choisis parmi les groupes ester, amide, sulfonamide, carbamate, thiocarbamate, urée, uréthane, thiourée, oxamido, guanidino, biguanidino et leurs combinaisons,
B) les polymères de polyamide comportant a) un squelette polymérique, ayant des motifs de répétition hydrocarbonés pourvus d'au moins un motif amide non pendant, et éventuellement b) au moins une chaîne grasse pendante et/ou au moins une chaîne grasse terminale éventuellement fonctionnalisées, comprenant au moins 4 atomes de carbone et étant liées à ces motifs hydrocarbonés,
C/ les copolymère d'oléfines,
D/ les organopolysiloxanes élastomériques,
E/ les polymères semi-cristallins,
F/ les esters de dextrine et d'acide gras,

et leurs mélanges

**7.** Composition selon la revendication précédente, dans laquelle le polymère siliconé comprend au moins un motif répondant à la formule :

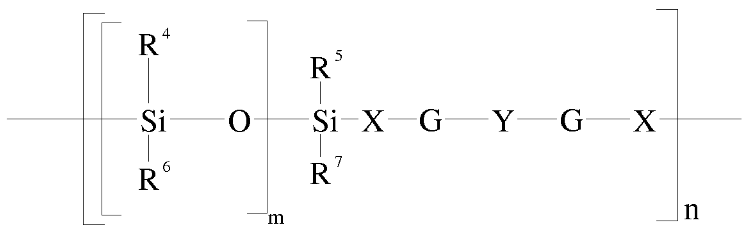

(I)

dans laquelle :

1) $R^4$, $R^5$, $R^6$ et $R^7$, identiques ou différents, représentent un groupe choisi parmi :

- les groupes hydrocarbonés, linéaires, ramifiés ou cycliques, en $C_1$ à $C_{40}$, saturés ou insaturés, pouvant contenir dans leur chaîne un ou plusieurs atomes d'oxygène, de soufre et/ou d'azote, et pouvant être substitués en partie ou totalement par des atomes de fluor,
- les groupes aryles en $C_6$ à $C_{10}$, éventuellement substitués par un ou plusieurs groupes alkyle en $C_1$ à $C_4$,
- les chaînes polyorganosiloxanes contenant ou non un ou plusieurs atomes d'oxygène, de soufre et/ou d'azote,

2) les X, identiques ou différents, représentent un groupe alkylène di-yle, linéaire ou ramifié en $C_1$ à $C_{30}$, pouvant contenir dans sa chaîne un ou plusieurs atomes d'oxygène et/ou d'azote,
3) Y est un groupe divalent alkylène linéaire ou ramifié, arylène, cycloalkylène, alkylarylène ou arylalkylène, saturé ou insaturé, en $C_1$ à $C_{50}$, pouvant comporter un ou plusieurs atomes d'oxygène, de soufre et/ou d'azote, et/ou porter comme substituant l'un des atomes ou groupes d'atomes suivants : fluor, hydroxy, cycloalkyle en $C_3$ à $C_8$, alkyle en $C_1$ à $C_{40}$, aryle en $C_5$ à $C_{10}$, phényle éventuellement substitué par 1 à 3 groupes alkyle en $C_1$ à $C_3$, hydroxyalkyle en $C_1$ à $C_3$ et amino alkyle en $C_1$ à $C_6$, ou
4) Y représente un groupe répondant à la formule :

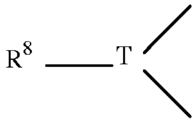

dans laquelle

- T représente un groupe hydrocarboné trivalent ou tétravalent, linéaire ou ramifié, saturé ou insaturé, en $C_3$ à $C_{24}$ éventuellement substitué par une chaîne polyorganosiloxane, et pouvant contenir un ou plusieurs atomes choisis parmi O, N et S, ou T représente un atome trivalent choisi parmi N, P et Al, et
- $R^8$ représente un groupe alkyle en $C_1$ à $C_{50}$, linéaire ou ramifié, ou une chaîne polyorganosiloxane, pouvant comporter un ou plusieurs groupes ester, amide, uréthane, thiocarbamate, urée, thiourée et/ou sulfonamide qui peut être lié ou non à une autre chaîne du polymère,

5) les G, identiques ou différents, représentent les groupes divalents choisis parmi :

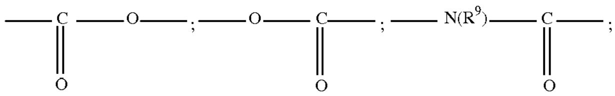

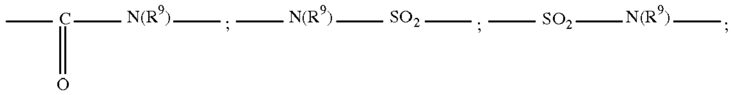

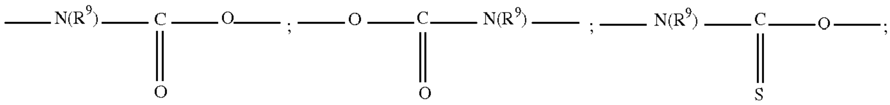

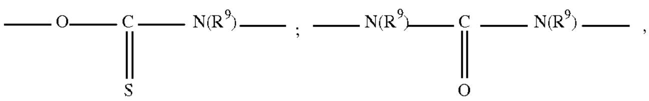

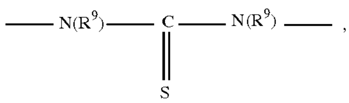

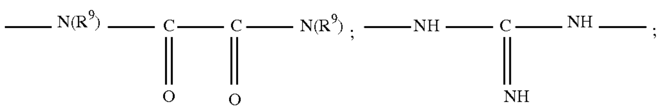

et

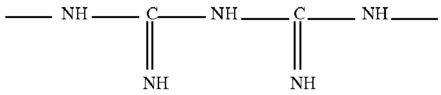

où $R^9$ représente un atome d'hydrogène ou un groupe alkyle, linéaire ou ramifié, en $C_1$ à $C_{20}$, à condition qu'au moins 50 % des $R^9$ du polymère représente un atome d'hydrogène et qu'au moins deux des groupes G du polymère soient un autre groupe que :

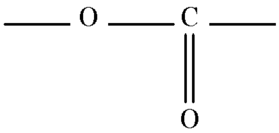 et 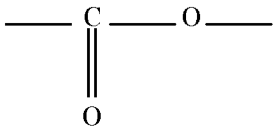 ;

6) n est un nombre entier allant de 2 à 500, de préférence de 2 à 200, et m est un nombre entier allant de 1 à 1000, de préférence de 1 à 700 et mieux encore de 6 à 200.

**8.** Composition selon l'une quelconque des revendications 6 ou 7, dans laquelle le polymère comprend au moins un motif de formule (III) ou (IV) :

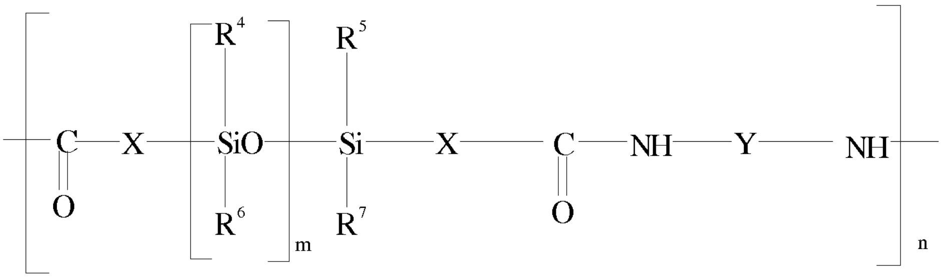

(III)

ou

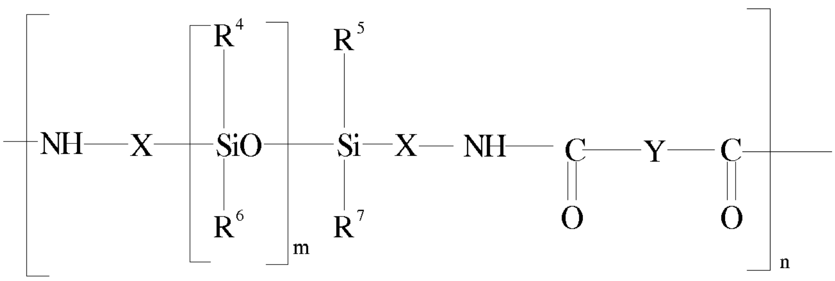

(IV)

dans lesquelles $R^4$, $R^5$, $R^6$, $R^7$, X, Y, m et n sont tels que définis dans la revendication 7.

**9.** Composition selon la revendication 6, **caractérisée par le fait que** le polyamide est choisi parmi les polyamides de formule (A) suivante :

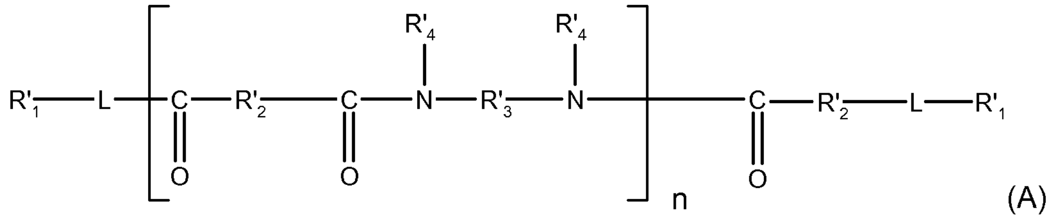

(A)

- n est un nombre entier allant de 1 à 30,
- $R'^1$ représente à chaque occurrence indépendamment une chaîne grasse et est choisi parmi un groupe alkyle ou alcényle ayant au moins 1 atome de carbone et notamment de 4 à 24 atomes de carbone ;
- $R'^2$, représente à chaque occurrence indépendamment un radical hydrocarboné comprenant de 1 à 52 atomes de carbone;
- $R'^3$, représente à chaque occurrence indépendamment un groupement organique comprenant au moins un atome choisi les atomes de carbone, d'hydrogène, d'azote, à la condition que $R'^3$ comprend au moins 3 atomes de carbone ;
- $R'^4$ représente à chaque occurrence indépendamment : un atome d'hydrogène, un groupe alkyle comprenant de 1 à 10 atomes de carbone ou une liaison directe à au moins un groupe choisi parmi $R'^3$ et un autre $R'^4$ de sorte que quand ledit groupe est un autre $R'^4$, l'atome d'azote auquel sont liés à la fois $R'^3$ et $R'^4$ fasse partie d'une structure hétérocyclique définie par $R'^4$-N-$R'^3$ à la condition que au moins 50% des $R'^4$ représentent un atome d'hydrogène, et
- L représente un groupe de liaison choisi parmi les groupes ester, éther, amine, urée, uréthane, thioester, thioéther, thiurée, thiouréthane, éventuellement substitué par au moins un groupe $R'^1$.

**10.** Composition selon l'une des revendications 6 ou 9, **caractérisée par le fait que** le polyamide est choisi parmi les polyamides de formule (B) suivante :

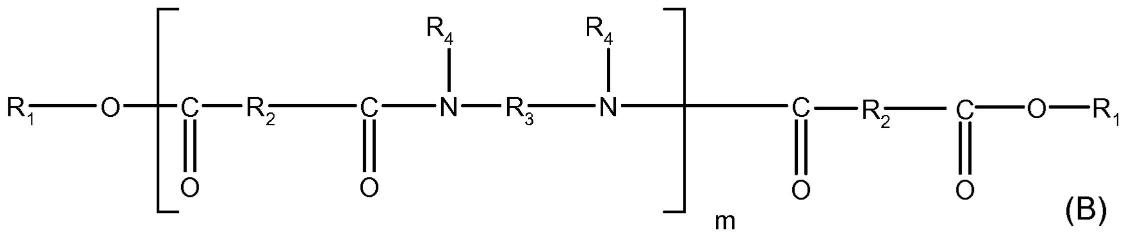

(B)

dans laquelle :

- m désigne un nombre entier de motifs amide tel que le nombre de groupes ester représente de 10 % à 50 % du nombre total des groupes ester et amide ;
- $R^1$ est à chaque occurrence indépendamment un groupe alkyle ou alcényle ayant au moins 4 atomes de carbone et notamment de 4 à 24 atomes de carbone ;
- $R^2$ représente à chaque occurrence indépendamment un groupe hydrocarboné en $C_4$ à $C_{42}$ à condition que 50 % des groupes $R^2$ représentent un groupe hydrocarboné en $C_{30}$ à $C_{42}$ ;
- $R^3$ représente à chaque occurrence indépendamment un groupe organique pourvu d'au moins 2 atomes de carbone, d'atomes d'hydrogène et optionnellement d'un ou plusieurs atomes d'oxygène ou d'azote ;
- et $R^4$ représente à chaque occurrence indépendamment un atome d'hydrogène, un groupe alkyle en $C_1$ à $C_{10}$ ou une liaison directe à $R^3$ ou à un autre $R^4$ de sorte que l'atome d'azote auquel sont liés à la fois $R^3$ et $R^4$ fasse partie d'une structure hétérocyclique définie par $R^4$-N-$R^3$, avec au moins 50 % des $R^4$ représentant un atome d'hydrogène.

**11.** Composition selon la revendication 6, **caractérisée en ce que** le copolymère est choisi parmi les copolymères formés par polymérisation de styrène et d'oléfine, l'oléfine étant choisie parmi l'éthylène, le propylène, le butadiène, l'isoprène.

**12.** Composition selon l'une des revendications 6 ou 11, **caractérisée en ce que** le copolymère d'oléfine est choisi parmi les copolymères, éventuellement hydrogéné, à blocs styrène et à blocs éthylène/alkylène en $C_3$-$C_4$.

**13.** Composition selon la revendication 6, **caractérisée en ce que** l'ester de dextrine et d'acide(s) gras répond à la formule (C) :

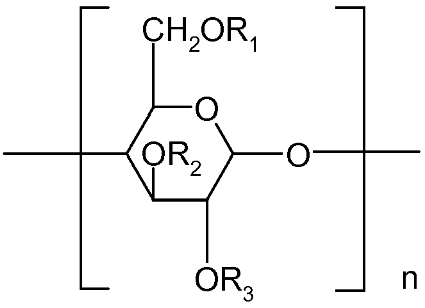

(C)

dans laquelle :
n est un entier allant de 3 à 200,
les radicaux $R_1$, $R_2$ et $R_3$, identiques ou différents, sont choisis parmi l'hydrogène ou un groupement acyle (R-CO-) dans lequel le radical R est un groupement hydrocarboné, linéaire ou ramifié, saturé ou insaturé, possédant de 5 à 29 atomes de carbone sous réserve qu'au moins un desdits radicaux $R_1$, $R_2$ ou $R_3$ est différent de l'hydrogène.

**14.** Composition selon l'une quelconque des revendications 6 ou 13, **caractérisée en ce que** l'ester de dextrine et d'acide(s) gras comprend au moins le palmitate de dextrine.

**15.** Composition selon l'une quelconque des revendications précédentes, **caractérisée en ce que** la phase huileuse continue comprend au moins une huile choisie parmi les huiles volatiles ou non volatiles.

**16.** Composition selon l'une quelconque des revendications précédentes, **caractérisé en ce qu'**elle ne comprend pas de cire.

**17.** Composition selon l'une quelconque des revendications 1 à 16, **caractérisé en ce que** qu'elle est anhydre.

**18.** Procédé de revêtement des matières kératiniques comprenant l'application sur lesdites matières kératiniques d'au moins une couche d'au moins une composition selon l'une quelconque des revendications 1 à 17.

**19.** Kit de maquillage et/ou de soin non thérapeutique des matières kératiniques comprenant :

- un récipient contenant une composition sous forme de mousse présentant une phase huileuse continue et au moins un agent structurant de la phase huileuse, ladite composition étant exempte de cire et
- un applicateur comportant au moins un élément d'application configuré pour appliquer la composition sur les matières kératiniques.

Office européen des brevets

# RAPPORT DE RECHERCHE EUROPEENNE

Numéro de la demande
EP 08 15 4891

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 7 175 836 B1 (HART KELLY DENISE [US] ET AL) 13 février 2007 (2007-02-13)<br>* colonne 11, ligne 62-64; exemples I-XII * | 1,18,19 | INV.<br>A61K8/06<br>A61Q1/00<br>A61Q19/00 |
| X | DATABASE WPI Week 199035<br>Thomson Scientific, London, GB; AN 1990-264448<br>XP002194121<br>& JP 02 184614 A (KOBAYASHI KOSE KK)<br>19 juillet 1990 (1990-07-19)<br>* abrégé * | 1,18,19 | |
| X | DATABASE WPI Week 199130<br>Thomson Scientific, London, GB; AN 1991-217654<br>XP002465139<br>& JP 03 133915 A (SHISEIDO CO LTD)<br>7 juin 1991 (1991-06-07)<br>* abrégé * | 1,18,19 | DOMAINES TECHNIQUES RECHERCHES (IPC) |
| X | FR 2 157 784 A (BRISTOL MYERS CO BRISTOL MYERS CO [US]) 8 juin 1973 (1973-06-08)<br>* page 10, ligne 24-36; revendication 1; exemples 1-13 * | 1,18,19 | A61K |
| X | EP 1 500 385 A (HAKUTO KK [JP])<br>26 janvier 2005 (2005-01-26)<br>* exemple 24 * | 1,18,19 | |

Le présent rapport a été établi pour toutes les revendications

| Lieu de la recherche | Date d'achèvement de la recherche | Examinateur |
|---|---|---|
| Munich | 8 juillet 2008 | Yon, Jean-Michel |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

2

EPO FORM 1503 03.82 (P04C02)

**ANNEXE AU RAPPORT DE RECHERCHE EUROPEENNE RELATIF A LA DEMANDE DE BREVET EUROPEEN NO.** EP 08 15 4891

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

08-07-2008

| Document brevet cité au rapport de recherche | Date de publication | Membre(s) de la famille de brevet(s) | Date de publication |
|---|---|---|---|
| US 7175836 B1 | 13-02-2007 | AU 2006331072 A1 | 05-07-2007 |
| | | CA 2615964 A1 | 05-07-2007 |
| | | WO 2007073878 A1 | 05-07-2007 |
| JP 2184614 A | 19-07-1990 | JP 2741230 B2 | 15-04-1998 |
| JP 3133915 A | 07-06-1991 | AUCUN | |
| FR 2157784 A | 08-06-1973 | AU 4236972 A | 22-11-1973 |
| | | CA 984257 A1 | 24-02-1976 |
| | | DE 2234316 A1 | 25-01-1973 |
| | | GB 1376649 A | 11-12-1974 |
| | | IT 1048985 B | 20-12-1980 |
| | | PH 10854 A | 13-09-1977 |
| | | SE 393395 B | 09-05-1977 |
| | | US 3770648 A | 06-11-1973 |
| | | ZA 7204689 A | 28-03-1973 |
| EP 1500385 A | 26-01-2005 | AU 2003221093 A1 | 13-10-2003 |
| | | WO 03082225 A1 | 09-10-2003 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 2006147390 A
- EP 847752 A
- US 5874069 A
- US 5919441 A
- US 6051216 A
- US 5981680 A
- WO 2003106614 A
- US 5783657 A
- US 6503522 B
- US 6399713 B
- US 3645705 A
- US 3148125 A
- US 6552160 B
- US 5500209 A
- US 5998570 A
- US 2002005562 A
- US 5221534 A
- EP 0295886 A
- US 5266321 A
- EP 1396259 A
- FR 2792190 A
- FR 2232303 A
- US 5162410 A
- WO 2004073626 A
- EP 1411069 A
- WO 04028488 A
- EP 749746 A
- WO 04055081 A